# EUROPÄISCHE PATENTSCHRIFT

(11) **EP 0 379 099 B1**
(45) Veröffentlichungstag und Bekanntmachung des Hinweises auf die Patenterteilung: **14.09.1994**
(21) Anmeldenummer: 90100659.3
(22) Anmeldetag: 13.01.1990
(51) Int. Cl.: C07D 231/56, A01N 43/56, C07D 405/12, C07D 409/12, A01N 43/08, A01N 43/10, A01N 43/16, A01N 43/18

(54) **Tetrahydroindazole mit Phenyletherstruktur**
Tetrahydroindazoles with a phenyl ether structure
Tétrahydroindazoles avec structures phényléther

(30) Priorität: 20.01.1989 DE 3901550
(43) Veröffentlichungstag der Anmeldung: 25.07.1990
(73) Patentinhaber: BASF Aktiengesellschaft, 67063 Ludwigshafen (DE)
(72) Erfinder: Rueb, Lothar, Dr., D-6720 Speyer (DE); Eicken, Karl, Dr., D-6706 Wachenheim (DE); Plath, Peter, Dr., D-6710 Frankenthal (DE); Westphalen, Karl-Otto, Dr., D-6720 Speyer (DE); Wuerzer, Bruno, Dr., D-6701 Otterstadt (DE)

(56) Entgegenhaltungen:
- EP-A- 0 105 721
- EP-A- 0 197 495
- US-A- 4 124 373
- US-A- 4 608 080
- PATENT ABSTRACTS OF JAPAN, Band 9, Nr. 21 (C-263)[1744], 29. Januar 1985;& JP-A-59 170 071
- PATENT ABSTRACTS OF JAPAN, Band 10, Nr. 194 (C-358)[2250], 8. Juli 1986;& JP-A-61 36 268 (MITSUBISHI CHEM. IND. LTD) 20-02-1986

## Beschreibung

Die vorliegende Erfindung betrifft neue N-(Phenyl)tetrahydroindazolderivate der allgemeinen Formel Ia und Ib 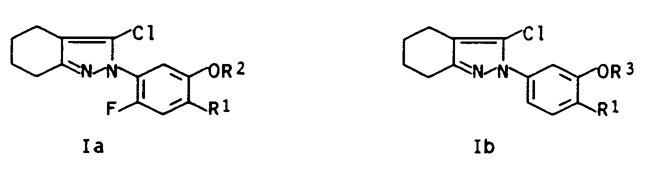
in denen die Substituenten folgende Bedeutung haben:
- R¹: Halogen
- R²: eine C₁-C₆-Alkoxycarbonyl-C₃-C₅-alkenylgruppe oder eine C₁-C₃-Alkylgruppe, welche in 1- oder 2-Position durch einen 5- oder 6-gliedrigen gesättigten oder einfach ungesättigten Heterocyclus substituiert ist, wobei dieser Heterocyclus ein Sauerstoff- oder Schwefelatom enthält und durch ein bis vier C₁-C₄-Alkylgruppen substituiert sein kann und
- R³: eine C₂-C₇-Alkylgruppe, eine C₃-C₆-Alkenylgruppe, eine C₃-C₆-Alkinylgruppe, eine C₁-C₆-Alkoxycarbonyl-C₃-C₅-alkenylgruppe oder eine C₁-C₃-Alkylgruppe, welche in 1- oder 2-Position durch eine C₁-C₆-Alkoxycarbonylgruppe oder einen 5- oder 6-gliedrigen gesättigten oder einfach ungesättigten Heterocyclus substituiert ist, wobei dieser Heterocyclus ein Sauerstoff- oder Schwefelatom enthält und durch ein bis vier C₁-C₄-Alkylgruppen substituiert sein kann.

Die Erfindung umfaßt auch sämtliche stereoisomeren Formen der Verbindungen Ia und Ib.

Außerdem betrifft die Erfindung die Herstellung der Verbindungen Ia und Ib, deren Verwendung als Herbizide sowie Mischungen zur Bekämpfung unerwünschten Pflanzenwuchses, welche diese Verbindungen Ia und Ib enthalten.

Die herbizide Wirkung von N-(Phenyl)tetrahydroindazolen ist aus der Literatur bekannt. So werden in der EP-A 105 721 beispielsweise Derivate der Formel I' 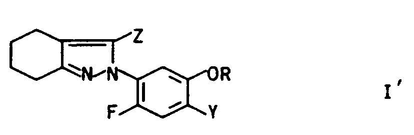
beschrieben, in der die Substituenten u.a. folgende Bedeutung haben:
- Y: Chlor oder Brom
- Z: Chlor oder Methyl und
- R: C₁-C₄-Alkyl, C₃-C₄-Alkenyl, C₃-C₄-Alkinyl, C₁-C₆-Alkoxycarbonylmethyl, C₃-C₆-Cycloalkoxycarbonylmethyl oder C₁-C₄-Halogenalkoxycarbonylmethyl.

Es werden jedoch Verbindungen gesucht, die bei geringeren Aufwandmengen eine bessere Wirkung gegen unerwünschte Pflanzen aufweisen und für Kulturpflanzen verträglich (selektiv) sind.

Entsprechend dieser Aufgabe wurden die eingangs definierten N-(Phenyl)tetrahydroindazolderivate Ia und Ib gefunden. Desweiteren wurden Verfahren zur Herstellung dieser Verbindungen sowie ihre Verwendung zur selektiven Bekämpfung unerwünschten Pflanzenwachstums gefunden.

Die erfindungsgemäßen N-(Phenyl)tetrahydroindazolderivate Ia und Ib sind auf verschiedenen Wegen zugänglich.

Man erhält sie beispielsweise dadurch, daß man ein entsprechend substituiertes N-(3-Hydroxylphenyl)tetrahydroindazol IIa und IIb in an sich bekannter Weise bei Temperaturen bis 200°C, vorzugsweise bei 25°C bis 150°C, in einem inerten organischen Lösungsmittel in Gegenwart einer Base mit einer entsprechenden Verbindung IIIa bzw. IIIb verethert 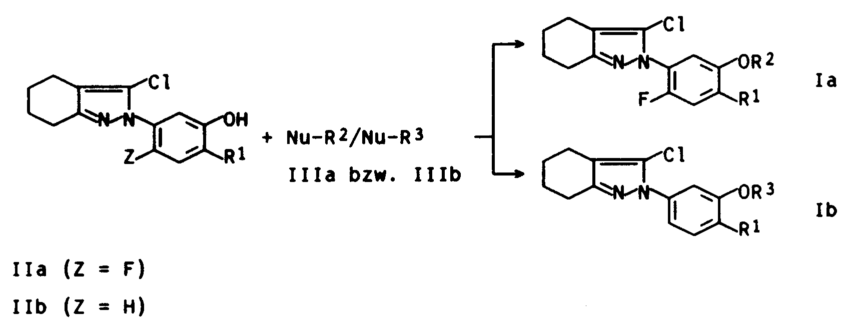
In den Formeln IIIa und IIIb bedeutet Nu eine übliche nucleophile Abgangsgruppe wie Halogen z.B. Chlor, Brom und Jod sowie Sulfonyloxygruppe z.B. Methylsulfonyloxy, Trifluorsulfonyloxy, Phenylsulfonyloxy und Tolylsulfonyloxy. Bevorzugt hat Nu die Bedeutung Chlor, Brom und Tolylsulfonyloxy.

Für die Umsetzung eignen sich besonders aprotisch polare Lösungsmittel wie Aceton, Acetonitril, Dimethylformamid, Dimethylsulfoxid, Tetrahydropyran und entsprechende Gemische.

Vorzugsweise führt man die Reaktion in Aceton, Acetonitril und Dimethylformamdid oder entsprechenden Gemischen durch.

Beispiele für üblicherweise eingesetzte Basen sind Natriumhydroxid, Kaliumhydroxid, Natrium- und Kaliumcarbonat, Natriummethylat und Natriumhydrid, wobei die Reaktion bevorzugt in Anwesenheit von kaliumcarbonat oder Natriumhydrid durchgeführt wird.

Die Herstellung der N-(3-Hydroxyphenyl)tetrahydroindazolderivate IIa ist in der Literatur bekannt (EP-A 105 721). Sie erfolgt in 4 Stufen ausgehend vom entsprechenden Nitrophenolderivat IVa gemäß dem folgenden Reaktionsschema. 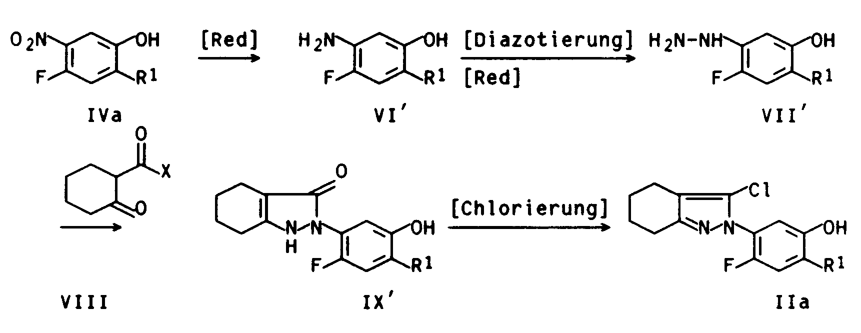
Auf ähnliche Weise erhält man die N-(3-Hydroxyphenyl)tetrahydroindazolderivate IIb in 6 Stufen, ausgehend von einem entsprechenden Nitrophenolderivat IVb, wobei als Zwischenstufe die Verbindungen Ib, worin R³ die Bedeutung C₁-C₄-Alkyl (Alk) besitzt, gebildet werden, gemäß dem folgenden Schema. 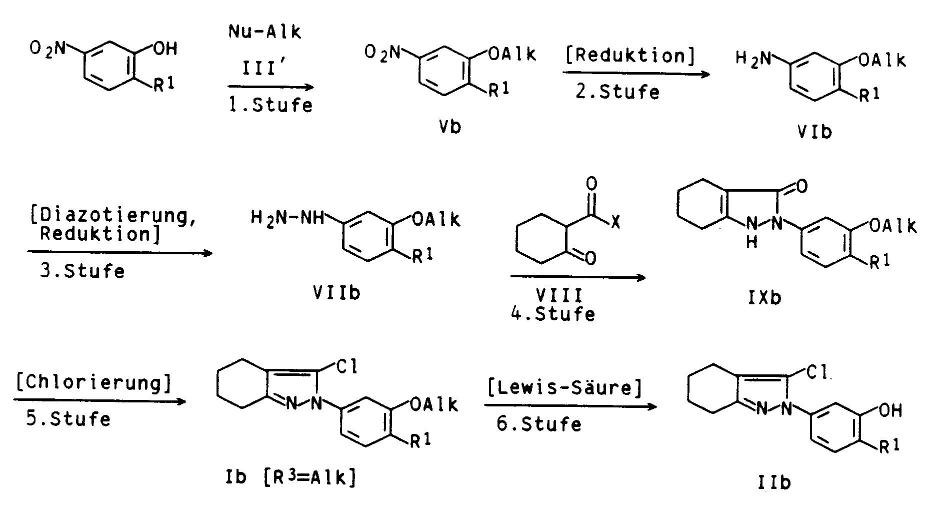
Zur Herstellung der Verbindungen Ib, in denen der Rest R³ unter den Chlorierungsbedingungen (5. Stufe) instabil ist, empfiehlt es sich, den Alkohol IIb mit der Verbindung IIIb analog den für die Umsetzung von IIa mit IIIa beschriebenen Bedindungen zu verethern. Verbindungen Ib, in denen R³ gegen diese Reaktionsbedingungen stabil ist, können analog dem vorstehenden Schema hergestellt werden.

Die einzelnen Reaktionsstufen sind an sich bekannt und werden nach allgemein üblichen Methoden durchgeführt.

Für die 1. Stufe (Veretherung) gelten im allgemeinen und im besonderen die gleichen Reaktionsbedingungen wie für die vorstehend geschilderte Umsetzung von IIa bzw. IIb mit IIIa bzw. IIIb.

Vorzugsweise wird diese Umsetzung in Aceton, Acetonitril und/oder Dimethylformamid bei Anwesenheit von Natriumhydrid oder Kaliumcarbonat als Base bei Temperaturen von 25 bis 150°C durchgeführt.

Die Reduktion der Nitrophenylether Vb zu den entsprechenden Anilinderivaten VIb wird nach allgemein üblichen Methoden, beispielsweise in Gegenwart von anorganischen Reduktionsmitteln wie Raney-Nickel, Eisen, Zinn-II-salzen in aprotisch oder protisch polaren Lösungsmitteln oder in Gegenwart von Wasserstoff an Edelmetallkontakten wie Platin und Palladium in aprotisch polaren Lösungsmitteln durchgeführt.

Das isolierte Anilinderivat IIb wird dann in einem inerten Lösungsmittel in an sich bekannter Weise bei Temperaturen von (-50)°C bis 50°C mit einem Nitrit diazotiert und anschließend in situ reduziert. 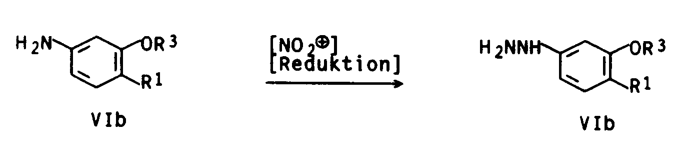
Sofern man für die Diazotierung ein anorganisches Nitrit wie salpetrige Säure und Alkalimetallnitrite verwendet, wird vorzugsweise in wäßriger Phase unter Zusatz von Mineralsäuren bei Temperaturen von -10°C bis 5°C gearbeitet, während die Diazotierung mit den üblichen organischen Nitriten wie Amylnitrit vorzugsweise in aprotisch polarer Lösung bei Temperaturen von -10°C bis 25°C durchgeführt wird.

Unabhängig von der Methode der Diazotierung kann die Reduktion zum entsprechenden Phenylhydrazin VIIb in situ erfolgen. Man versetzt dazu die erhaltene Reaktionsmischung bei -20°C bis 25°C mit einem anorganischen Reduktionsmittel wie einem Zinn-II-salz, einem Alkalimetallsulfit, einem Alkalimetallhydrogensulfit und Schwefeldioxid in Substanz oder in Lösung.

Das so erhaltene Phenylhydrazinderivat VIIb wird anschließend in einem inerten organischen Lösungsmittel bei Temperaturen bis 200°C, vorzugsweise 25°C bis 150°C in Anwesenheit oder Abwesenheit einer Base mit einem Cyclohexanonderivat VIII cyclisiert. 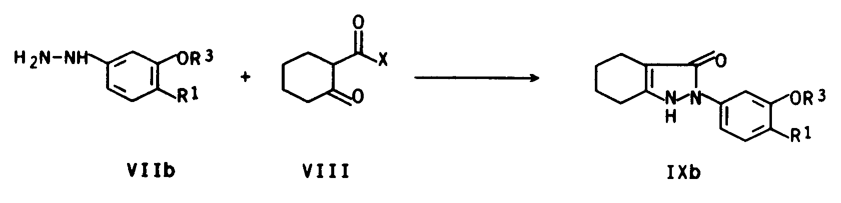
Die Gruppe X in Formel VIII bedeutet eine nucleophile Abgangsgruppe. Es kommen hier beispielsweise Halogenatome wie Chlor und Brom, Alkoxygruppen wie Methoxy, Ethoxy, Propyloxy und Isopropyloxy und Sulfonate wie Tosylat in Betracht.

Die Reaktion wird je nach der Bedeutung von X in protischen oder aprotisch polaren oder aprotisch unpolaren Lösungsmitteln in Anwesenheit oder Abwesenheit einer Base durchgeführt.

Sofern X ein Halogenatom bedeutet, arbeitet man vorzugsweise in Gegenwart einer Base in einem aprotisch polaren oder aprotisch unpolaren Lösungsmittel.

Als Basen eignen sich hier besonders tertiäre Amine wie Triethylamin, Diisopropylethylamin, N,N-Dimethylanilin, N,N-Dimethyl-p-aminopyridin, Pyridin, Isochinolin, N-Methylpyrolidin, N,N,N'N'-Tetramethylethylendiamin, 1,5-Diazabicyclo(4,3,0)non-5-en(DBN) und 1,8-Diazabicyclo(5,4,0)undec-7-en(DBU).

Geeignete Lösungsmittel sind beispielsweise Toluol, Xylol, Chloroform und Tetrahydrofuran sowie entsprechende Gemische.

Vorzugsweise arbeitet man in Toluol oder Chloroform in Anwesenheit von Triethylamin, Pyridin oder DBU als Base.

Sofern X ein Alkoholat oder Sulfonat bedeutet, sind protisch polare Lösungsmittel wie insbesondere Eisessig bevorzugt. Die Reaktion wird in diesem Fall ohne Zusatz einer Base durchgeführt.

Die Chlorierung des so erhaltenen Indazolderivates IXb zum N-(Phenyl)tetrahydroindazolderivat Ib (R³ = Alkyl) erfolgt in an sich bekannter Weise mit einem allgemein üblichen Chlorierungsreagens. 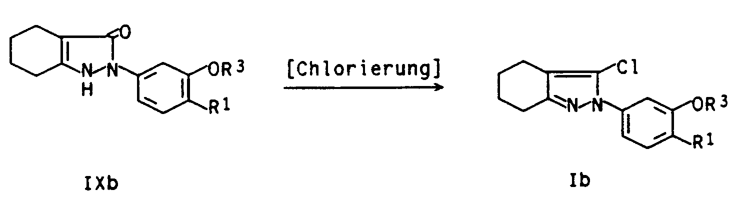
Diese Umsetzung verläuft bei Temperaturen bis 200°C , vorzugsweise 50 bis 180°C in Anwesenheit eines inerten organischen Lösungsmittels sowie in Anwesenheit oder Abwesenheit einer Base.

Geeignete Chlorierungsmittel sind z.B. Oxychloride wie Phosphoroxytrichlorid, Thionylchlorid, Phosgen oder Trichlormethylchloroformiat sowie Chloride wie Phosphortrichlorid, Phosphorpentachlorid oder Schwefeltetrachorid. Vorzugsweise verwendet man Phosphoroxytrichlorid.

Geeignete Lösungsmittel sind beispielsweise Toluol, Xylol, Chloroform und Methylenchlorid, insbesondere Toluol und Chloroform.

Als Basen kommen die obengenannten tertiären Amine in Betracht.

Besonders bevorzugt ist die Umsetzung mit Phosphoroxytrichlorid ohne Lösungsmittelzusatz sowie die Umsetzung mit Trichlormethylchloroformiat in Toluol oder Chloroform als Lösungsmittel.

Aus diesen erfindungsgemäßen N-(Phenyl)tetrahydroindazolderivaten Ib, in denen R³ eine C₁-C₄-Alkylgruppe (Alk) bedeutet, sind die Derivate Ib in denen R³ eine unter den vorstehend beschriebenen Bedingungen instabile Gruppe ist, auf allgemein üblichen Wegen über Etherspaltung und anschließende Veretherung zugänglich. 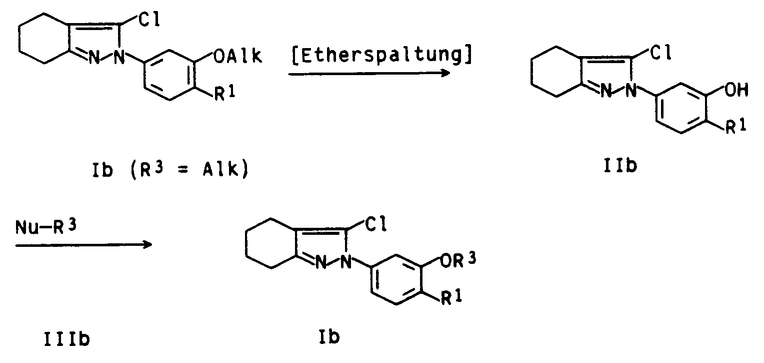
Zur Etherspaltung setzt man das N-(Alkoxyphenyl)tetrahydroindazolderivat Ib (R³ = Alk) in an sich bekannter Weise in einem inerten organischen Lösungsmittel bei Temperaturen von 0°C bis 150°C mit einer Lewis-Säure um.

Besonders geeignete Lewis-Säuren sind hier beispielsweise Bortrifluorid und sein Etherat, Aluminiumtrichlorid, Phosphortrichlorid und Bortribromid.

Als Lösungsmittel werden Ether wie Tetrahydrofuran, Halogenkohlenwasserstoffe wie Chloroform sowie Kohlenwasserstoffe wie Toluol und entsprechende Gemische bevorzugt.

Die anschließende Umsetzung des N-(3-Hydroxyphenyl)tetrahydroindazols IIb mit dem nucleophilen Reagens IIb erfolgt analog zu den vorstehend für die Umsetzung von IIa und IIIa geschilderten Bedingungen.

Im Hinblick auf die bestimmungsgemäße Verwendung der Verbindungen Ia und Ib kommen als Substituenten folgende Reste in Betracht:
- R¹: Halogen wie Fluor, Chlor und Brom, insbesondere Chlor;
- R²: Alkoxycarbonylalkenyl wie 2-(Methoxycarbonyl)propyl-2-enyl, 2-(Ethoxycarbonyl)prop-2-enyl, 2-(Propyloxycarbonyl)-prop-2-enyl, 2-(i-Propyloxycarbonyl)prop-2-enyl, 2-(Methoxycarbonyl)but-2-enyl, 2-(Ethoxycarbonyl)but-2-enyl, 2-(Propyloxycarbonyl)but-2-enyl, 2-(i-Propyloxycarbonyl)but-2-enyl;
Alkyl wie Methyl, Ethyl, Propyl und 1-Methylethyl, vorzugsweise Methyl und Ethyl, welches in 1- oder 2-Position durch einen 5- oder 6-gliedrigen gesättigten oder einfach ungesättigten Heterocyclus, enthaltend ein Sauerstoff- oder Schwefelatom substituiert ist, wie Tetrahydrofuranyl, Tetrahydrothienyl, 2,3-Dihydrofuranyl, 2,5-Dihydrofuranyl, 2,3-Dihydrothienyl, 2,5-Dihydrothienyl, Tetrahydropyranyl, Tetrahydrothiopyranyl, 2H-3,4-Dihydropyranyl, 2H-5,6-Dihydropyranyl, 2H-3,4-Dihydrothiopyranyl und 2H-5,6-Dihydrothiopyranyl, vorzugsweise Tetrahydrofuranyl, Tetrahydrothienyl, Tetrahydropyranyl, Tetrahydrothiopyranyl, 2H-3,4-Dihydropyranyl, 2H-5,6-Dihydropyranyl, 2H-3,4-Dihydrothiopyranyl und 2H-5,6-Dihydrothiopyranyl, wobei diese Ringe durch bis zu 4 der unter R² genannten Alkylgruppen, vorzugsweise durch eine bis vier Methylgruppen substituiert sein können; im Fall von substiuierten Ringen können alle möglichen sterischen Anordnungen vorkommen;
- R³: Alkyl wie Ethyl, Propyl, 1-Methylethyl, Butyl, 1-Methylpropyl, 2-Methylpropyl und 1,1-Dimethylethyl, Pentyl, 1-Methylbutyl, 2-Methylbutyl, 3-Methylbutyl,1,2-Dimethylpropyl, 1,1-Dimethylpropyl, 2,2-Dimethylpropyl, 1-Ethylpropyl, n-Hexyl, 1-Methyl-pentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl und 1-Ethyl-2-methylpropyl, sowie die isomeren Heptylreste;
Alkenyl wie 1-Propenyl, 2-Propenyl, 1-Methylethenyl, 1-Butenyl, 2-Butenyl, 3-Butenyl, 1-Methyl-1-propenyl, 1-Methyl-2-propenyl, 2-Methyl-1-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,1-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
Alkinyl wie 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-3-butinyl, 2-Methyl-3-butinyl, 1-Methyl-2-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Alkinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
oder ein Rest R² der im allgemeinen und im besonderen die oben gegebene Bedeutung besitzt.

Spezielle Beispiele besonders aktiver Verbindungen Ia und Ib sind in den nachstehenden Tabellen A, B und C aufgeführt.

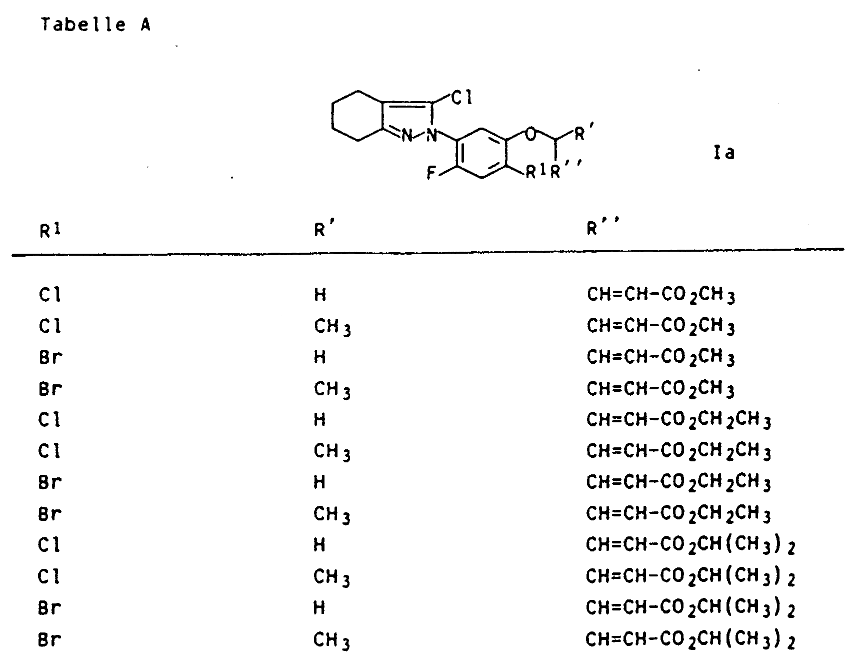

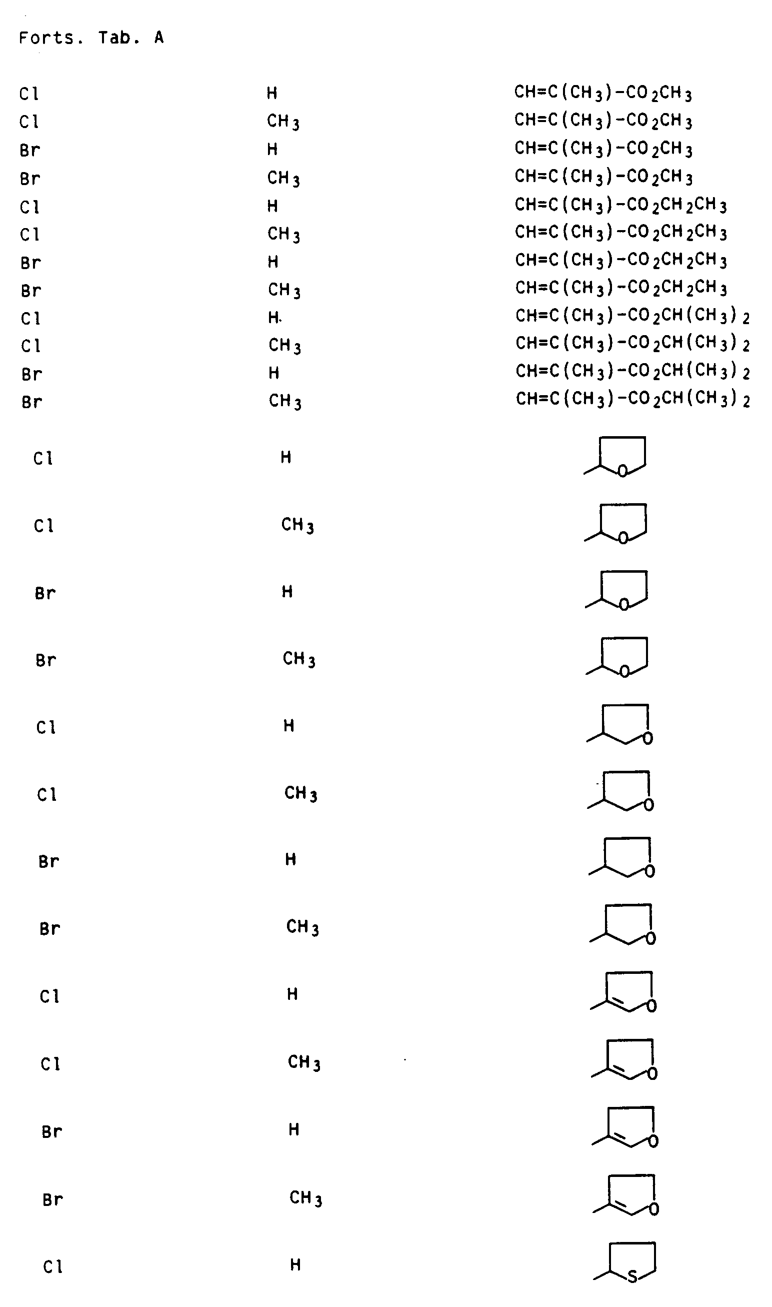

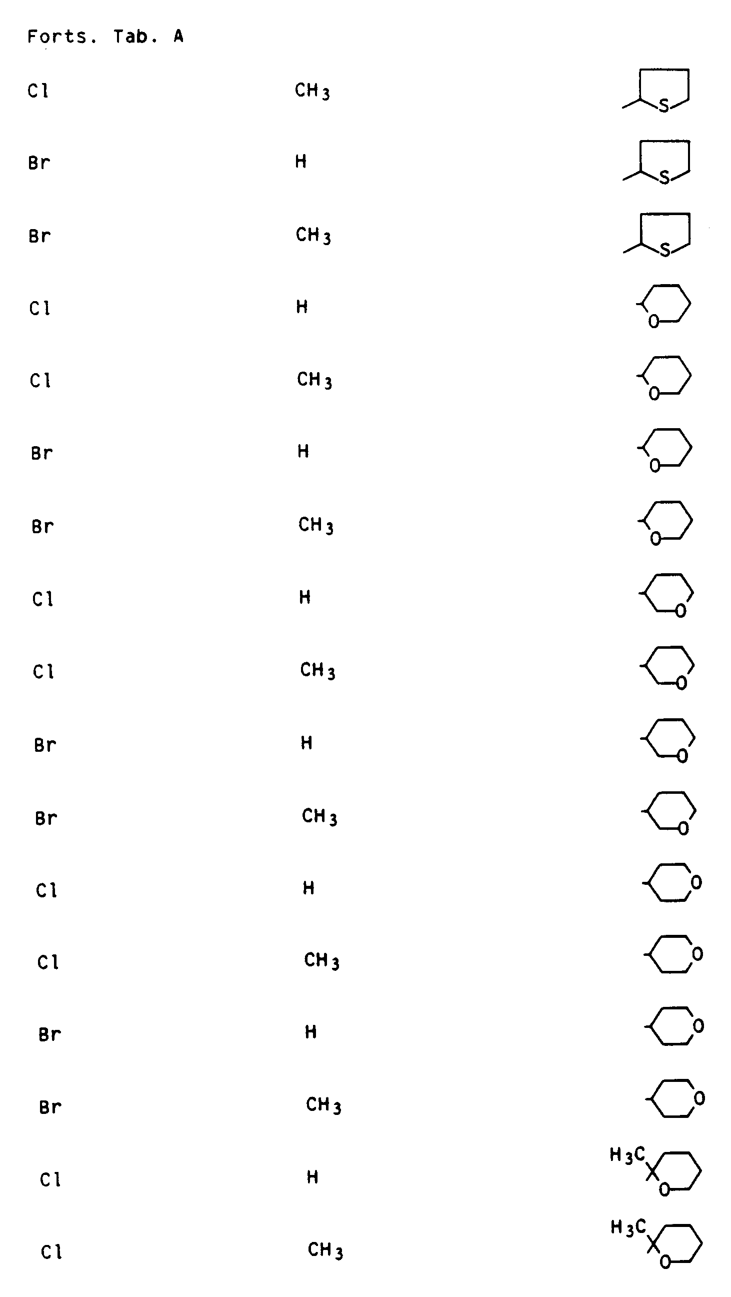

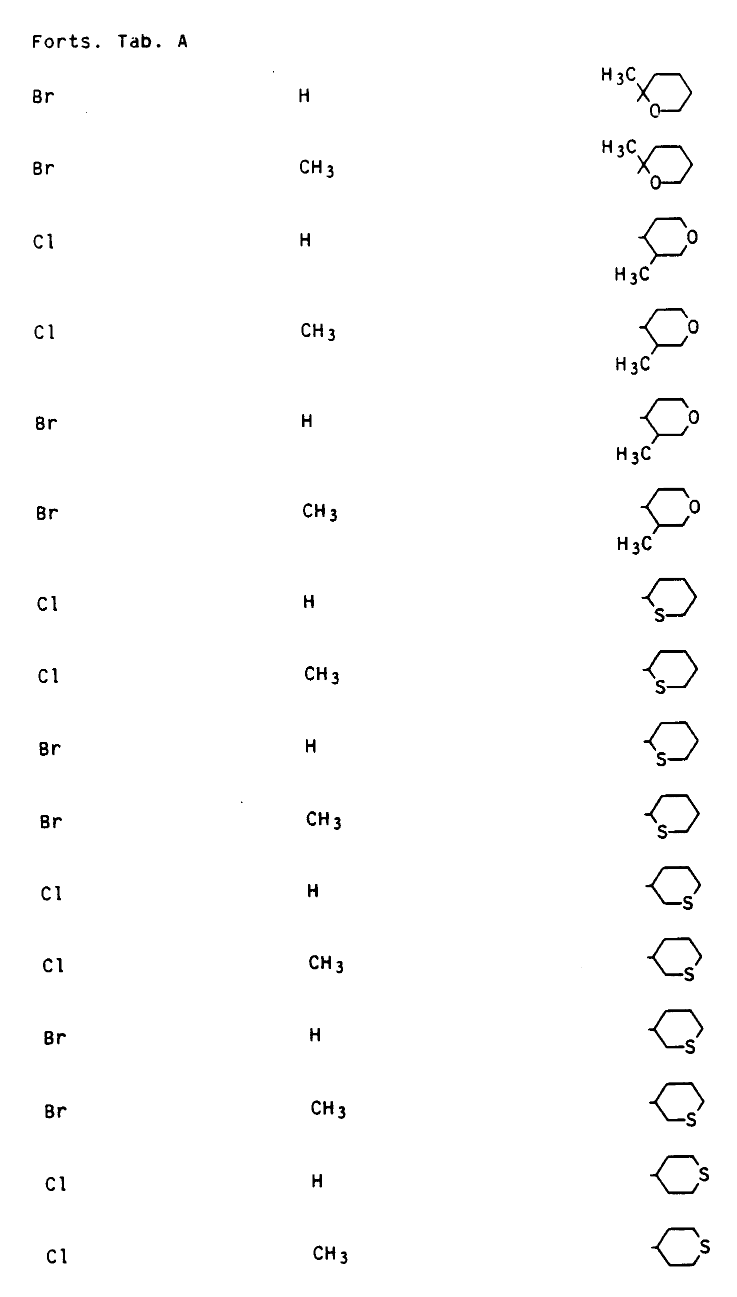

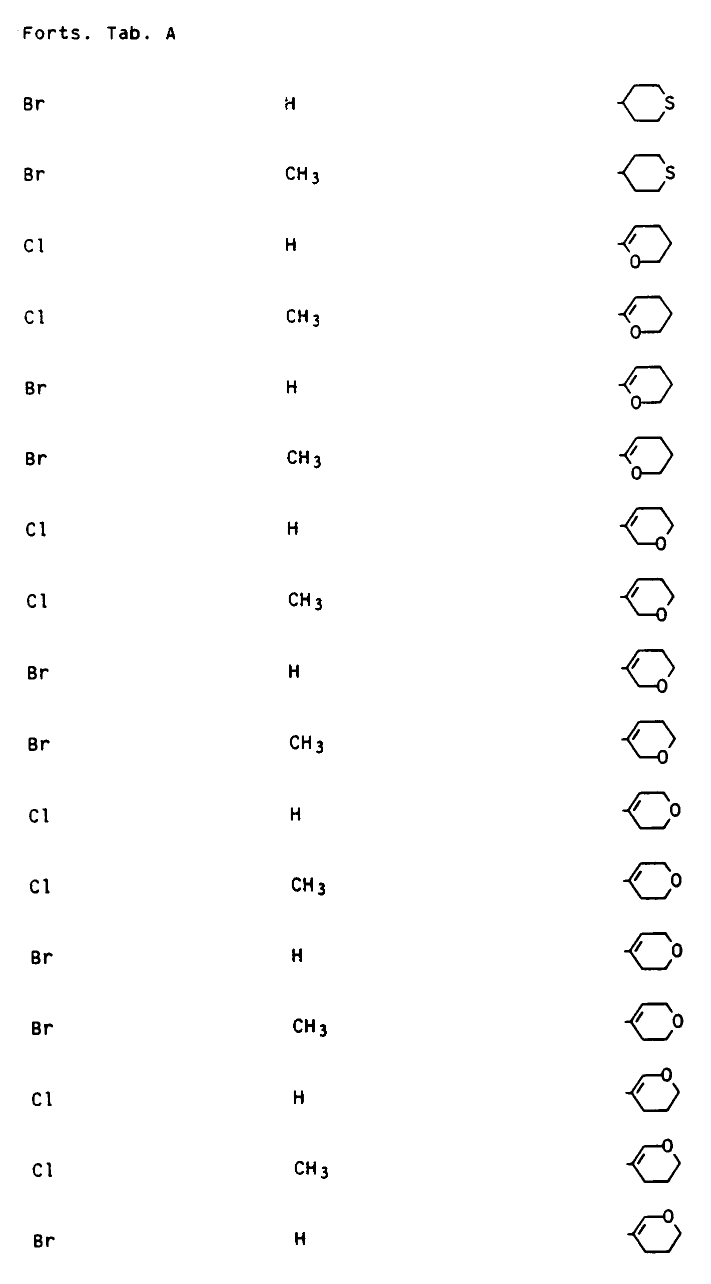

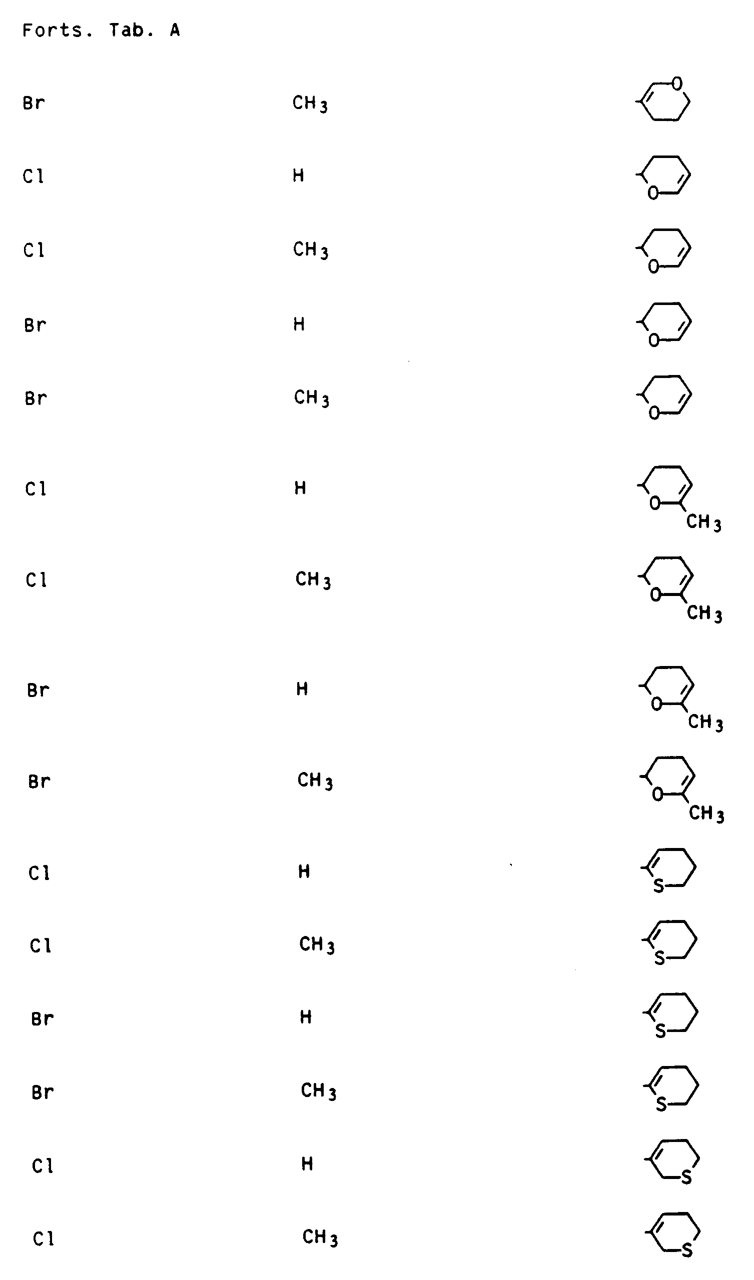

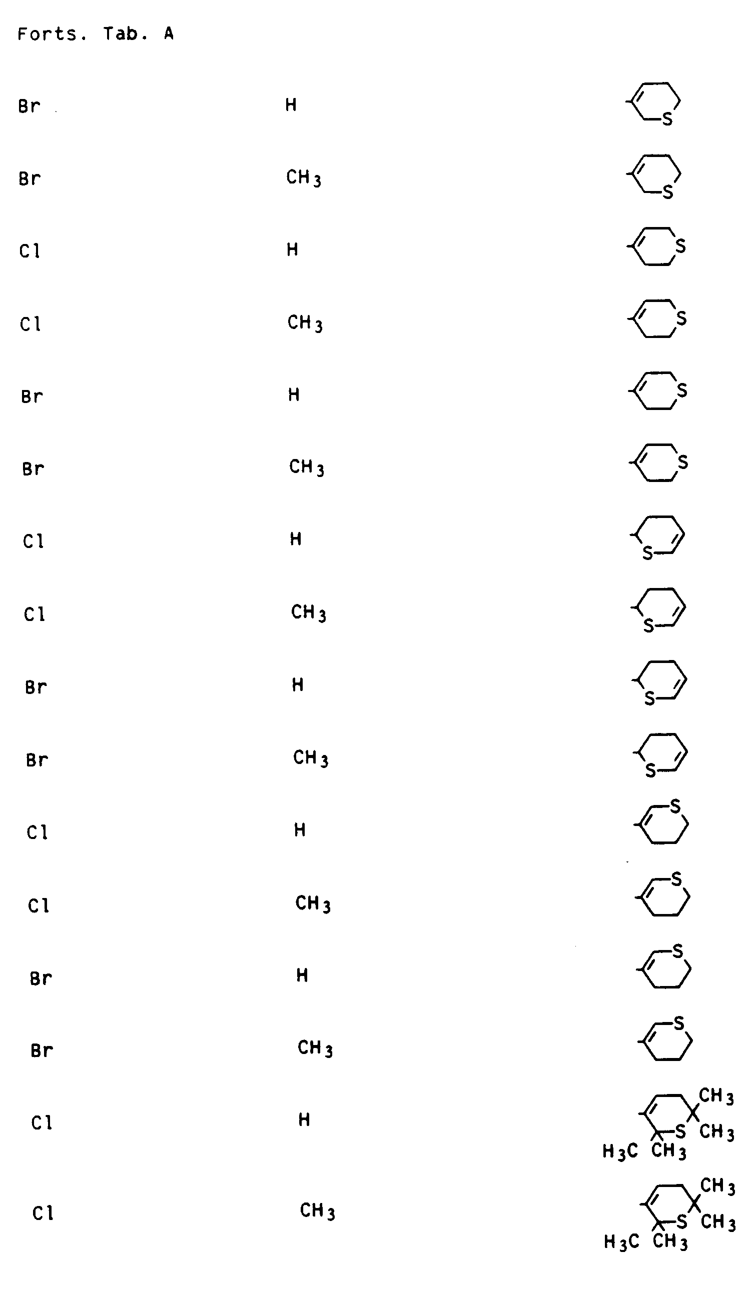

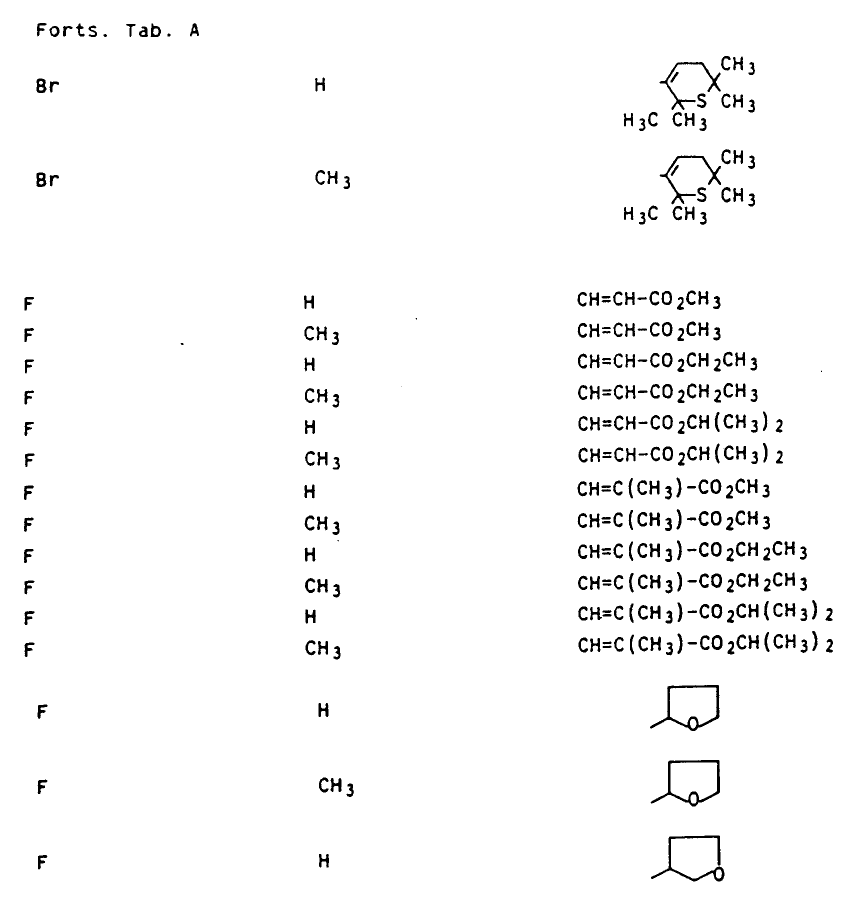

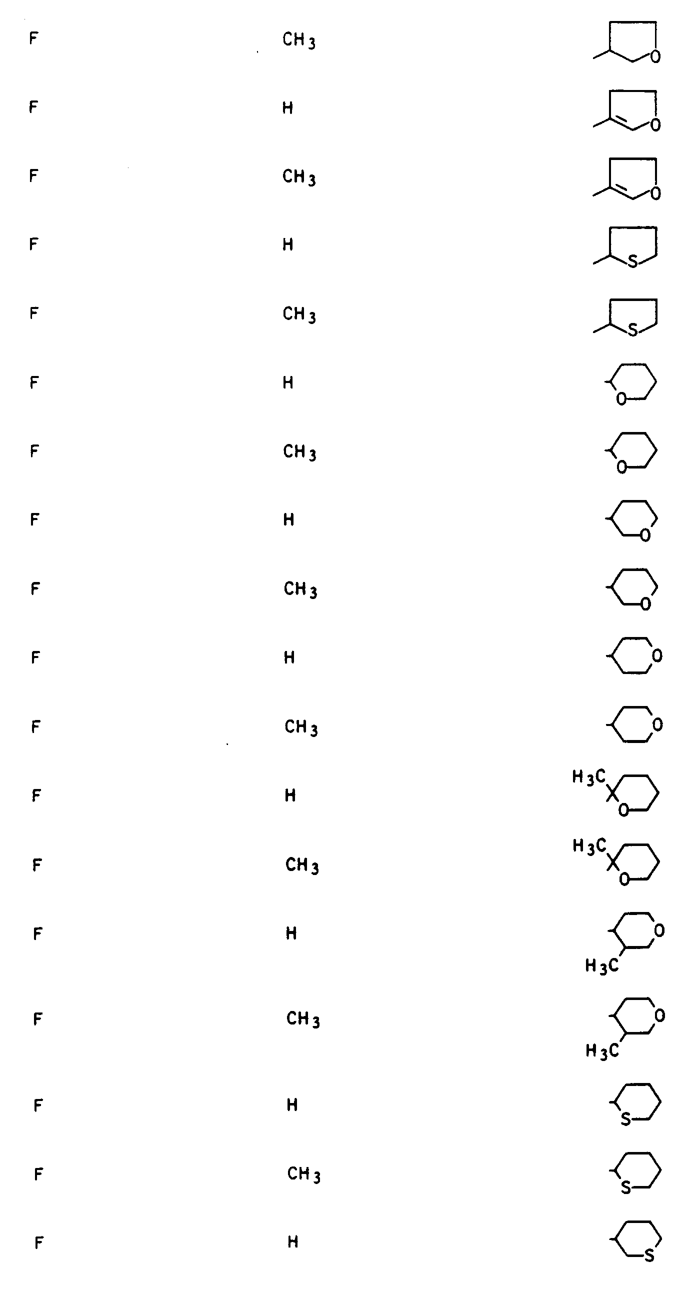

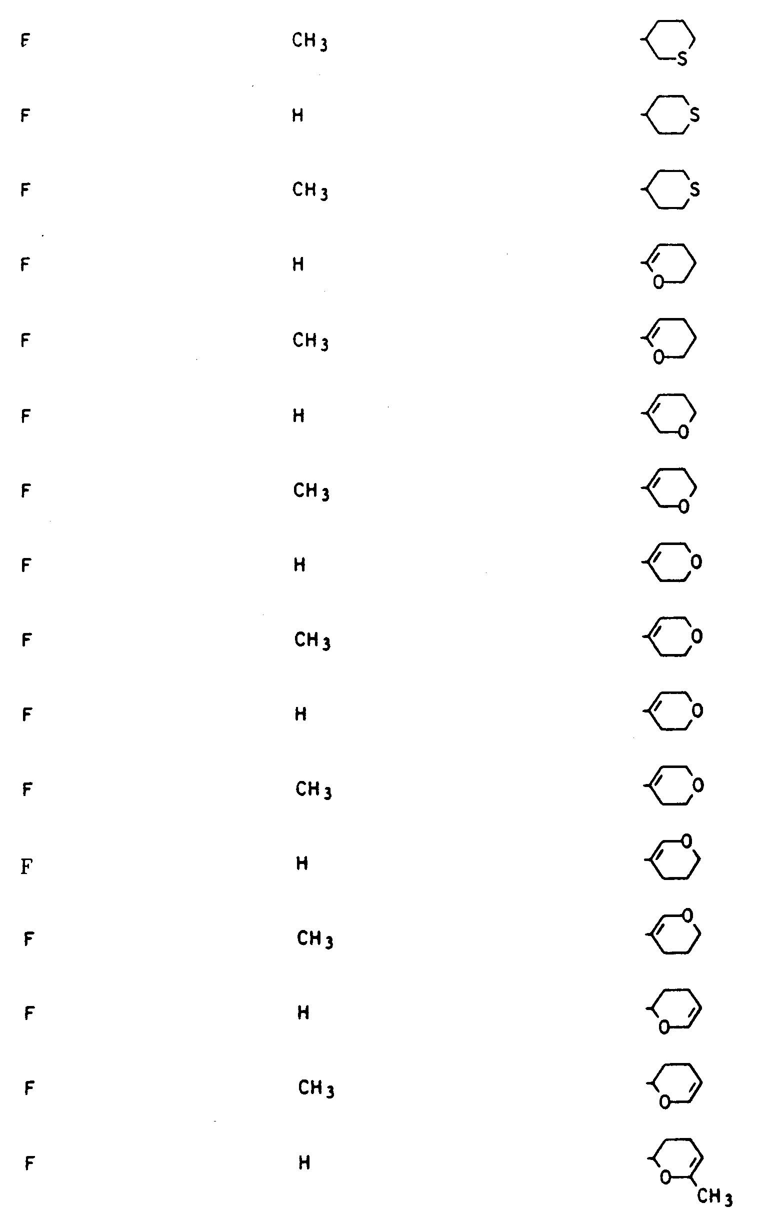

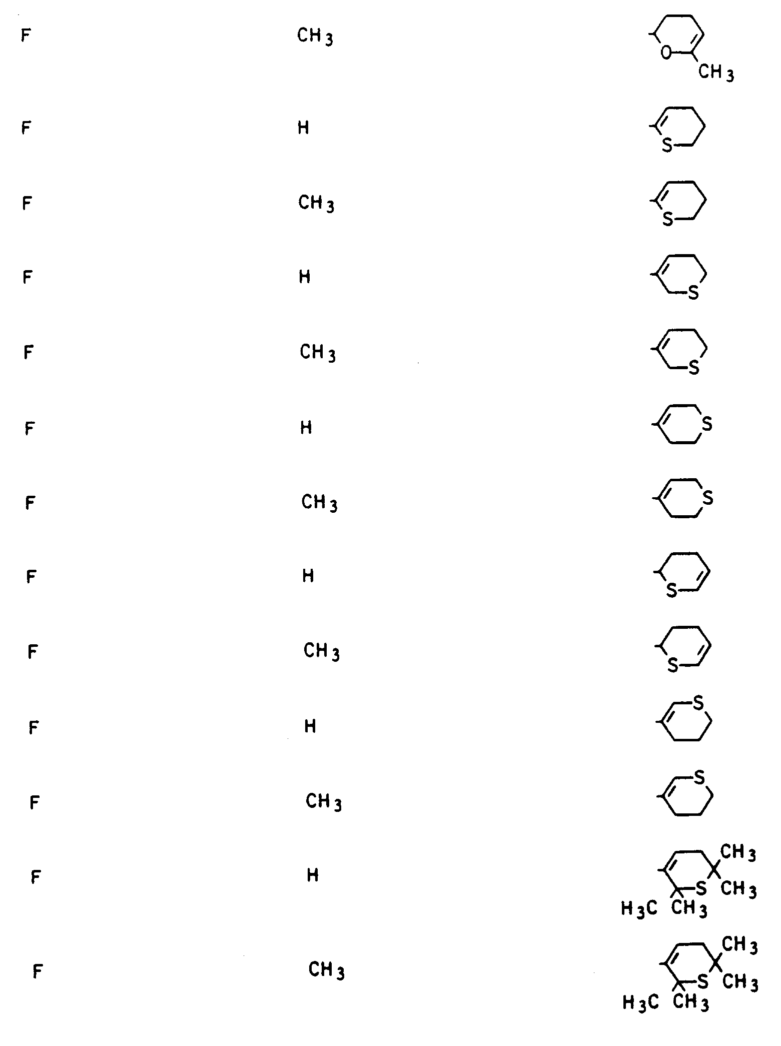

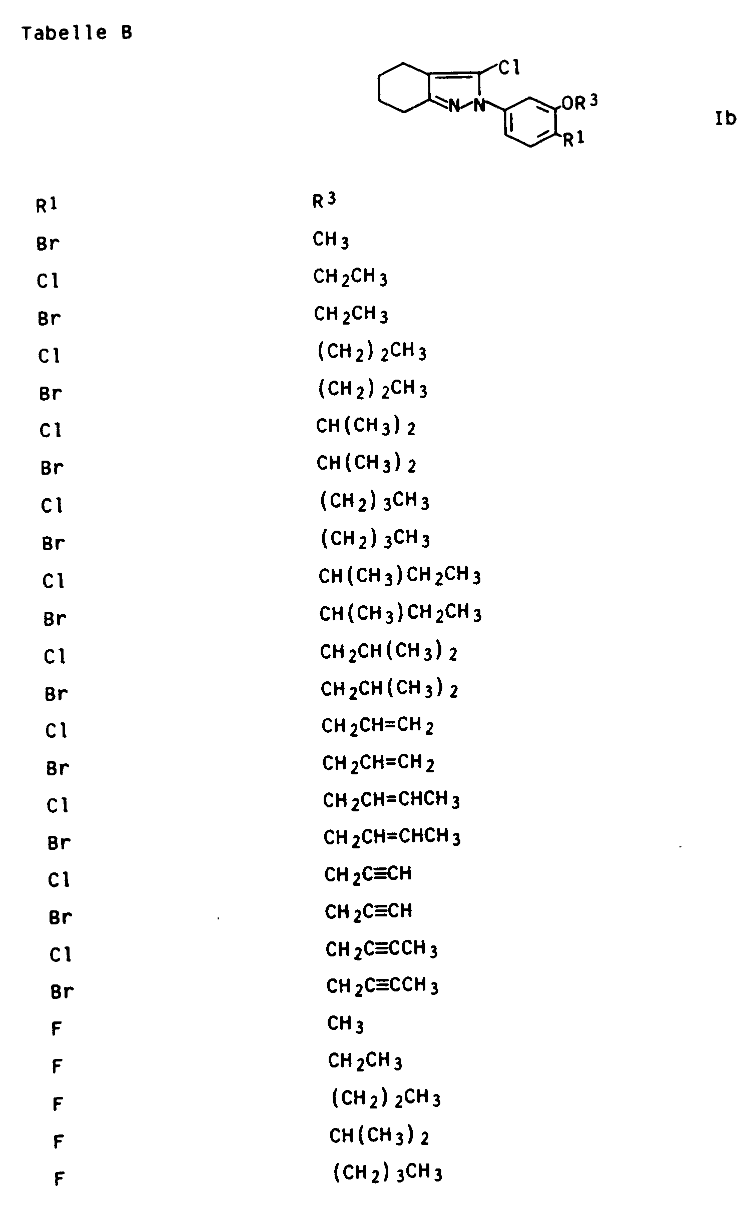

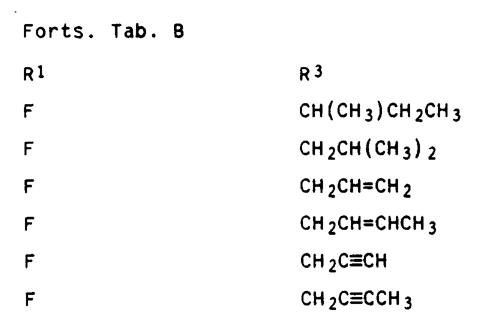

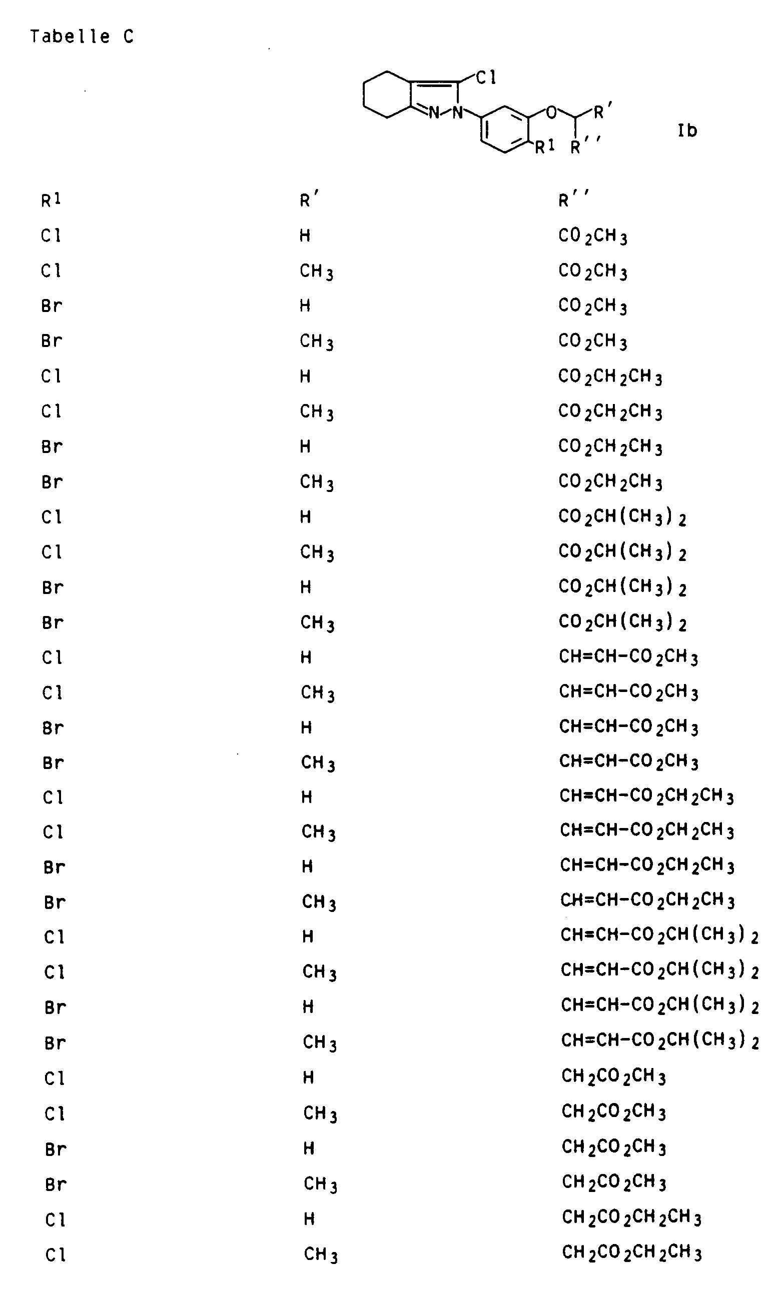

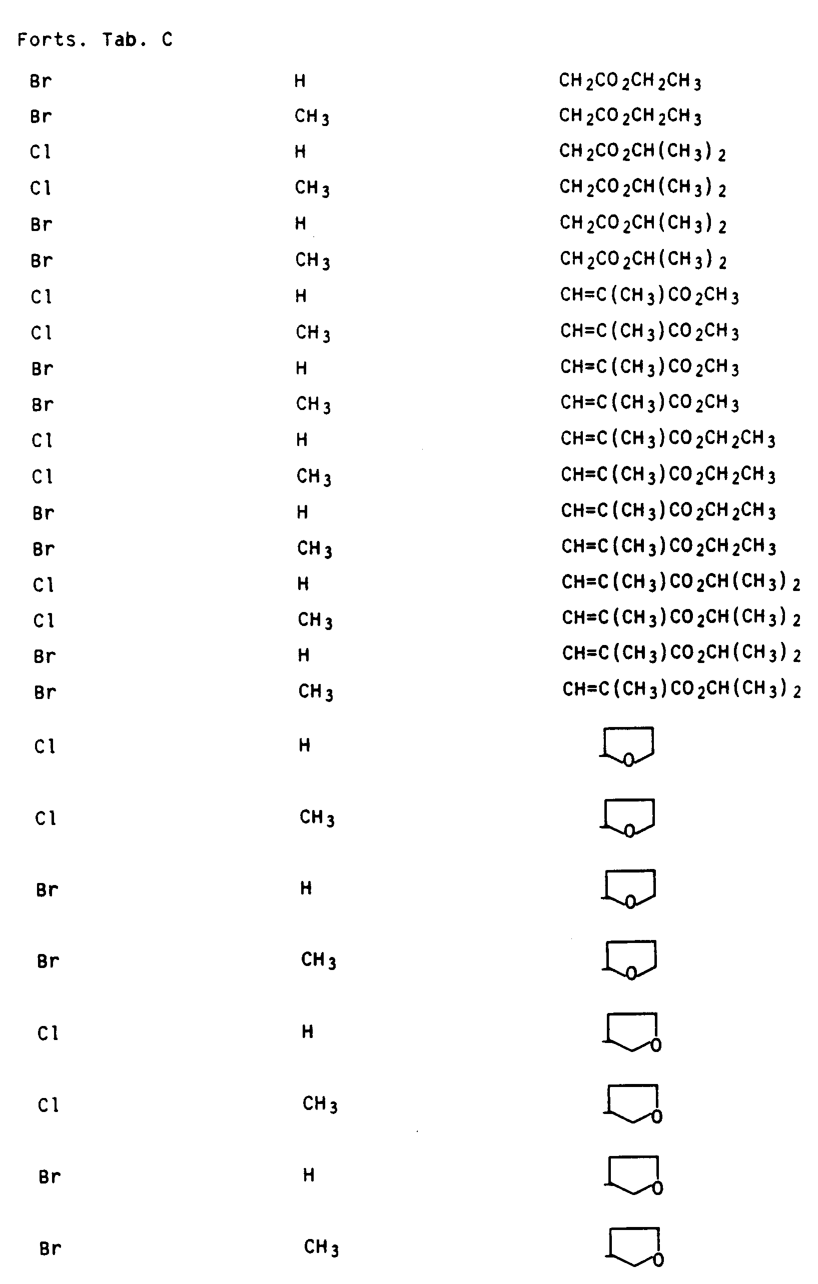

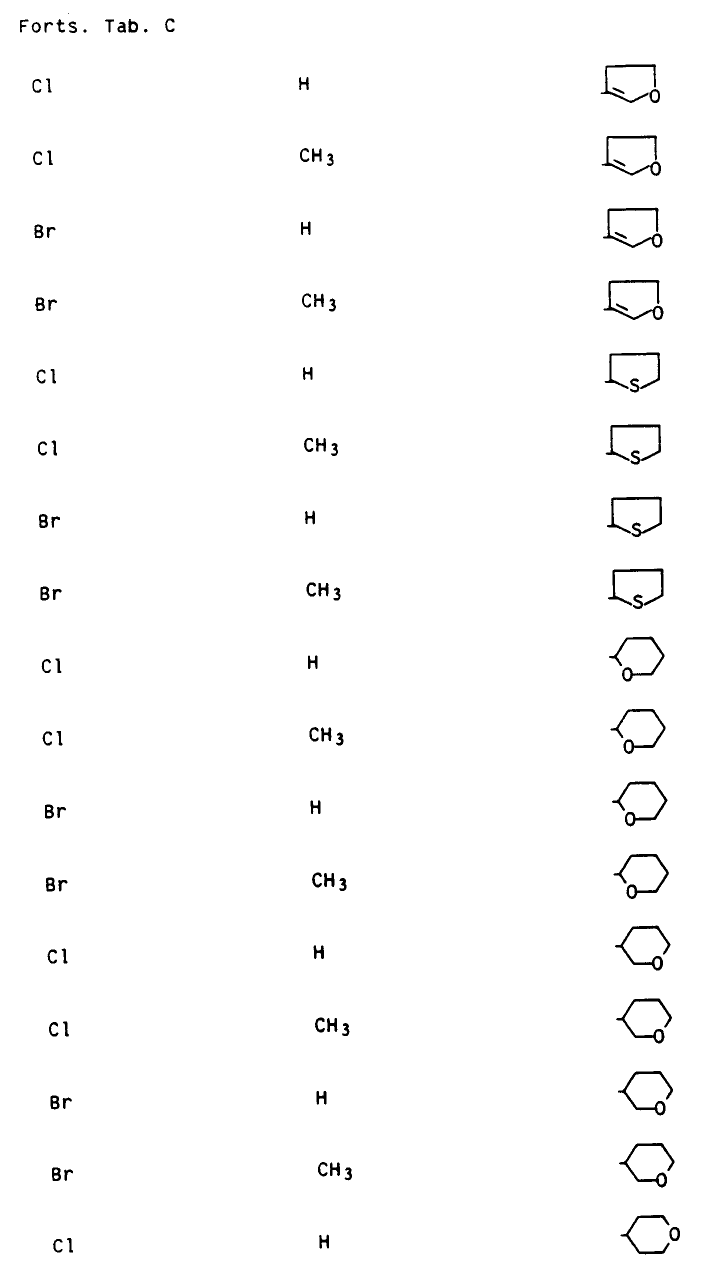

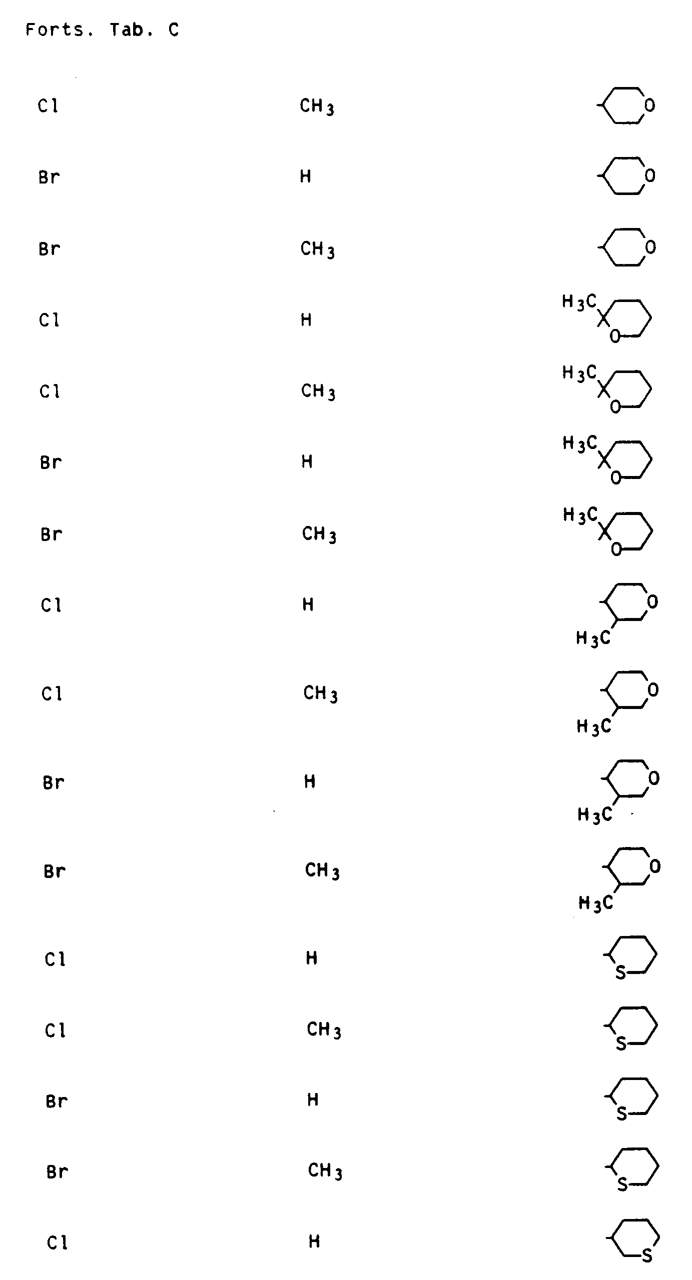

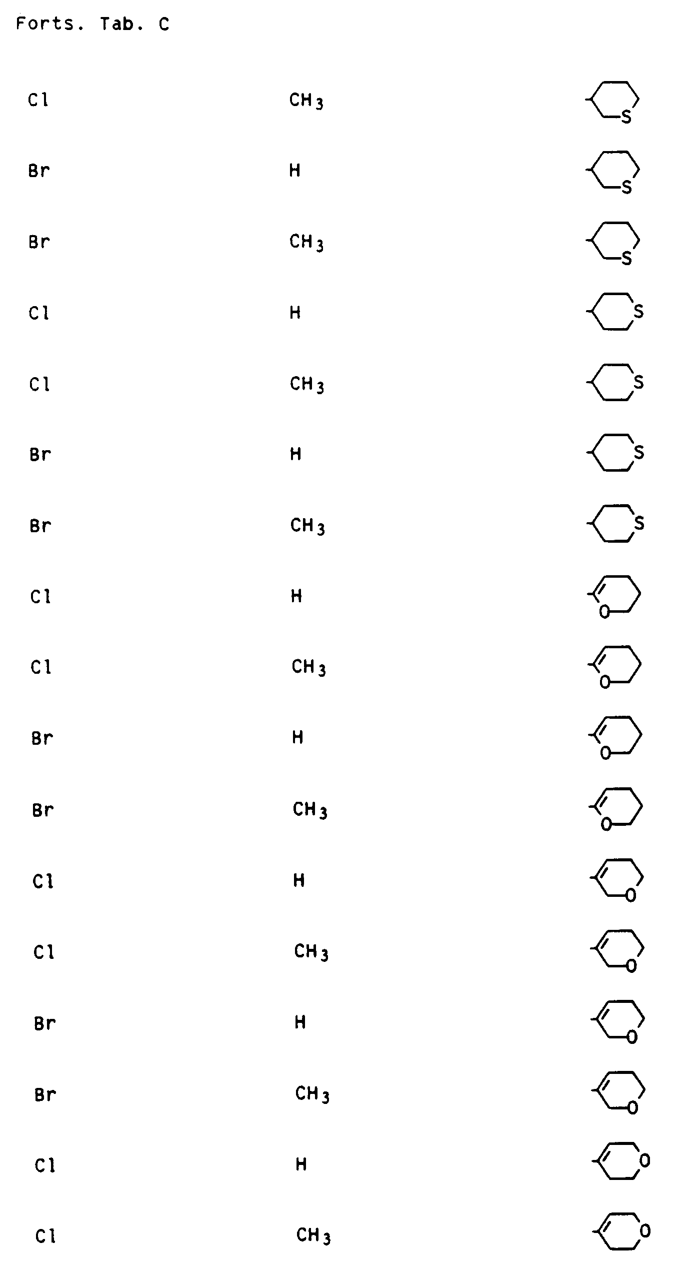

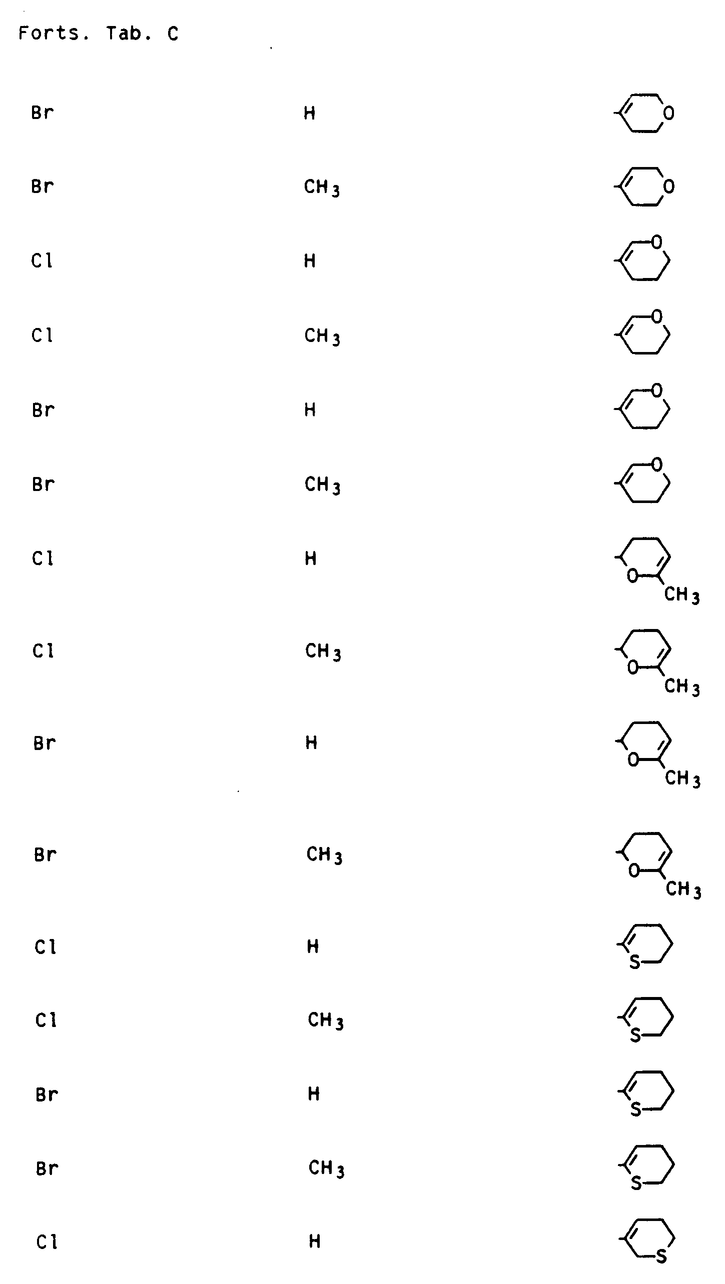

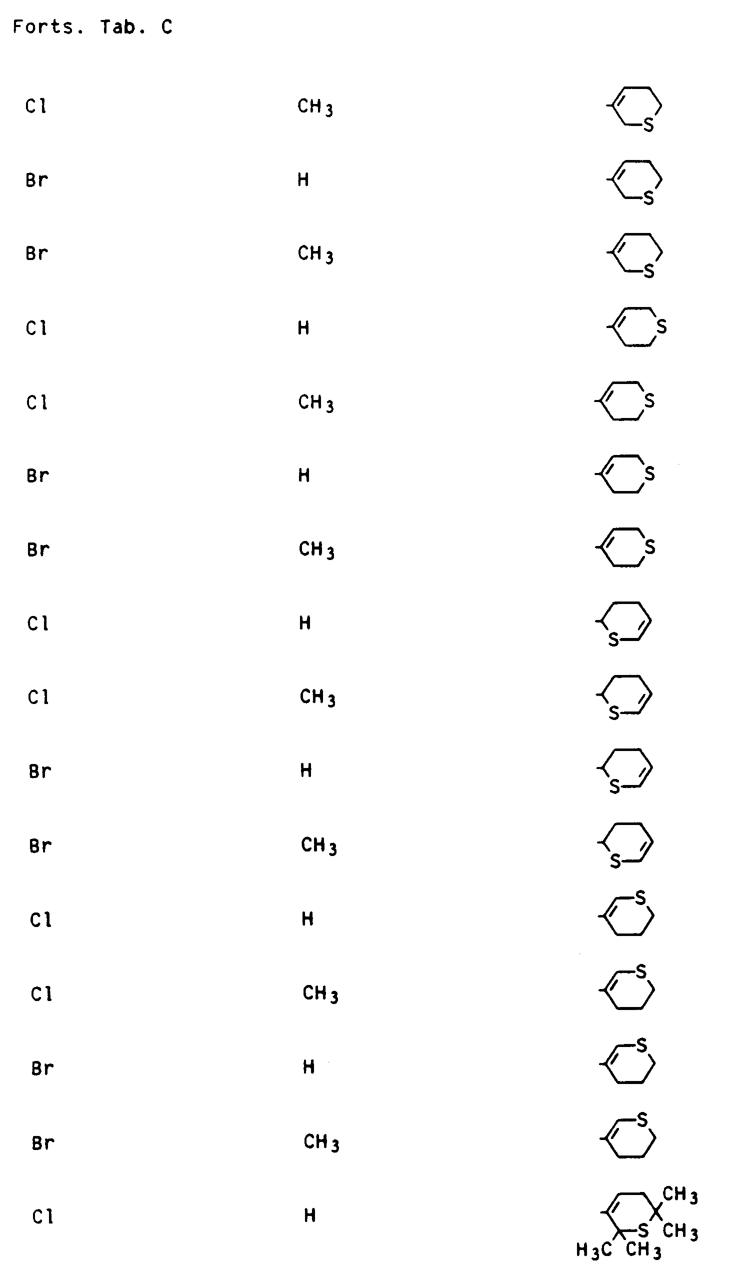

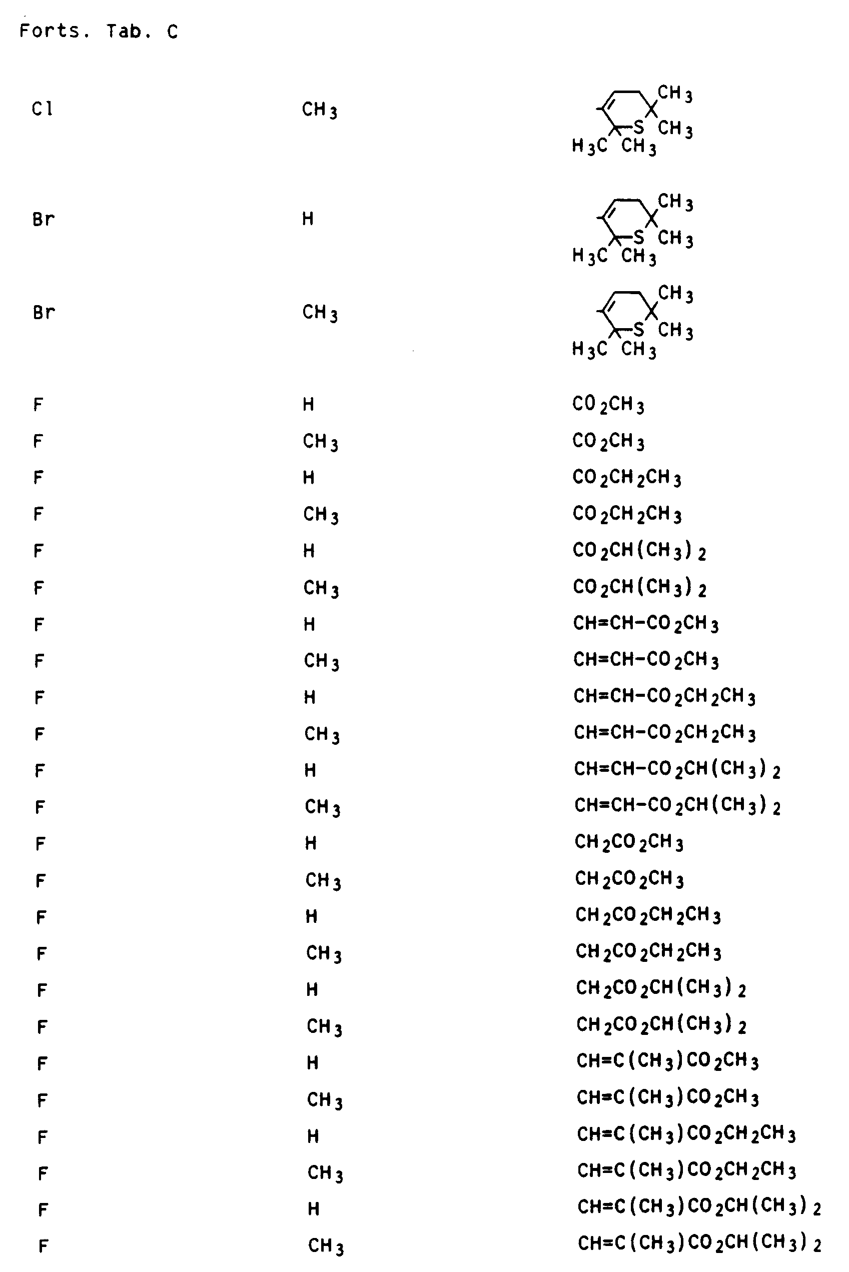

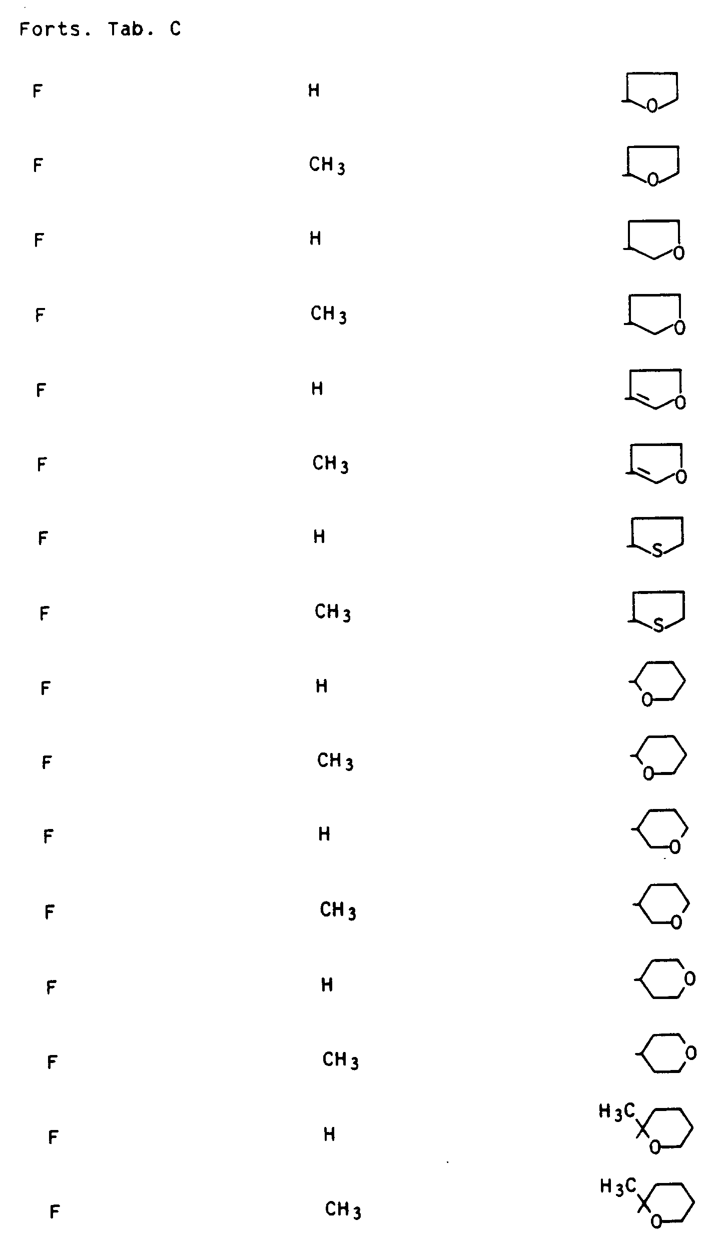

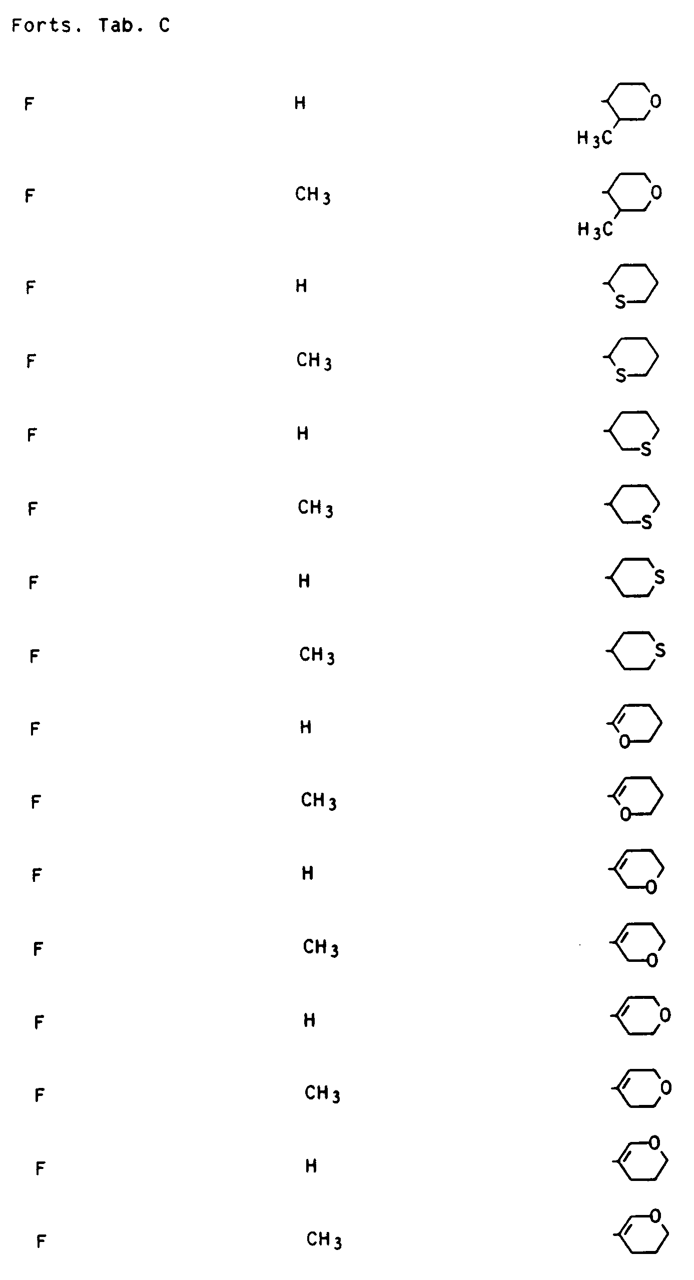

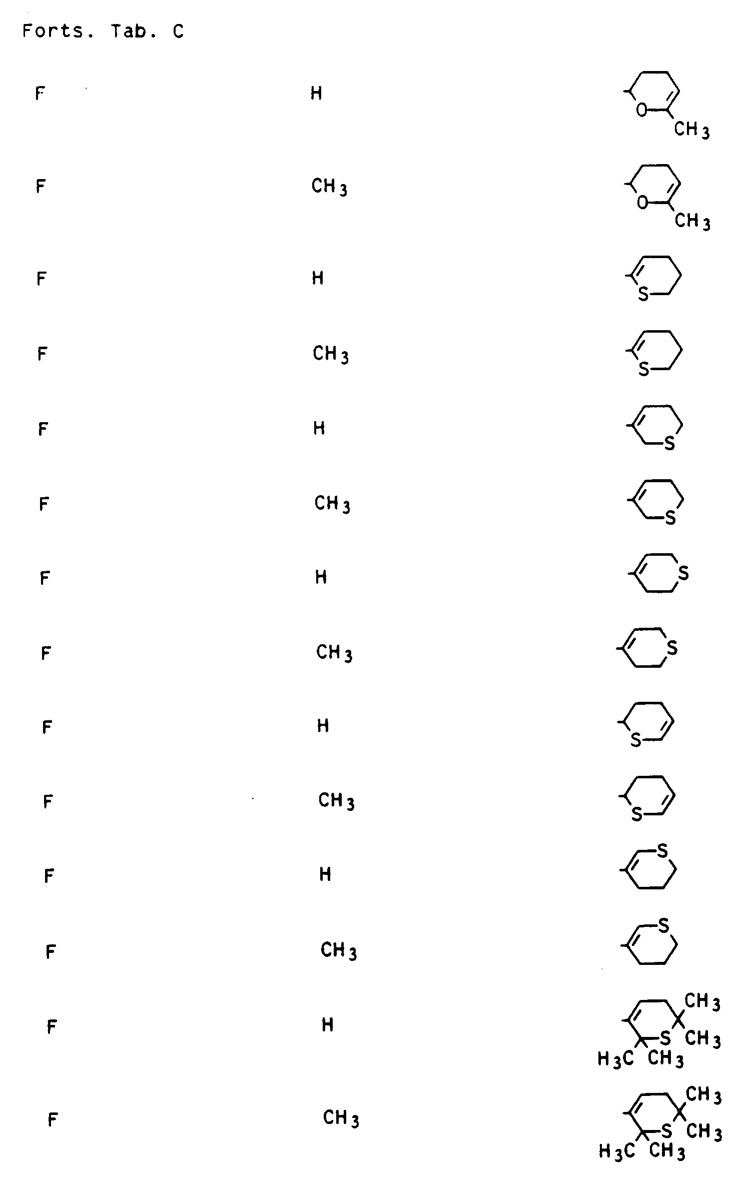

Die N-Phenyltetrahydroindazolderivate Ia und Ib bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen Ia und Ib eignen sich allgemein zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxyid, N-Methylpyrrolidon oder Wasser.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphtholinsulfonsäure, sowie von Fettsäuren, Alkyl-und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Napthalin und seiner Derivate mit Formaldehyd, Kondensationsprodukte des Naphthalins bzw. der Naphthalinsulfunsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettolkoholethylenoxid-Kondensate, ethoxyliertes Rizinusöl, Polyoxyethlenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, Z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoff und pflanzliche Produkte, wie Getreidemehl, Baumrinden-, Holz und Nußschalenmehl, Cellulosepulver oder andere feste Trägerstoffe.

Die Formulierungen enthalten zwischen 0,1 und 95 Gew.-%, vorzugsweise zwischen 0,5 und 90 Gew.-%, Wirkstoff.

Beispiele für Formulierungen sind:
- I.: Man vermischt 90 Gew.-Teile der Verbindung Nr. 1.001 mit 10 Gew.-Teilen N-Methyl-α-pyrrolidon und erhält eine Lösung, die zur Anwendung in Form kleinster Tropfen geeignet ist.
- II.: 20 Gew.-Teile der Verbindung Nr. 1.004 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecyclbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in 100 000 Gew.-Teilen Wasser erhält man eine wäßrige Dispersion, die 0,02 Gew.-% des Wirkstoffs enthält.
- III.: 20 Gew.-Teile der Verbindung Nr. 1.006 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion.
- IV.: 20 Gew.-Teile des Wirkstoffs Nr. 1.001 werden in einer Mischung gelöst, die aus 25 Gew.-Teilen Cyclohexanon, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser enthält man eine wäßrige Dispersion, die 0,02 Gew.-% des Wirkstoffs enthält.
- V.: 20 Gew.-Teile des Wirkstoffs Nr. 1.002 werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.-% des Wirkstoffs enthält.
- VI.: 3 Gew.-Teile des Wirkstoffs Nr. 1.005 werden mit 97 Gew.-Teilen feinteiligem Kaolin vermischt. Man erhält auf diese Weise ein Stäubemittel, das 3 Gew.-% des Wirkstoffs enthält.
- VII.: 30 Gew.-.Teile des Wirkstoffs Nr. 1.003 werden mit einer Mischung aus 92 Gew.-Teilen pulverförmigen Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
- VIII.: 20 Gew.-Teile des Wirkstoffs Nr. 1.004 werden mit 2 Gew.-Teilen Calciumsalz der Dodecylbenzolsulfonsäure, 8 Gew.-Teilen Fettalkohol-polyglykolether, 2 Gew.-Teilen Natriumsalz eines Phenol-Harnstoff-Formaldehyd-Kondensates und 68 Gew.-Teilen eines paraffinischen Mineralöls innig vermischt. Man erhält eine stabile ölige Dispersion.

Die Applikation kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (postdirected, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 0,5 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen werden. In Betracht kommen beispielsweise folgende Kulturen:

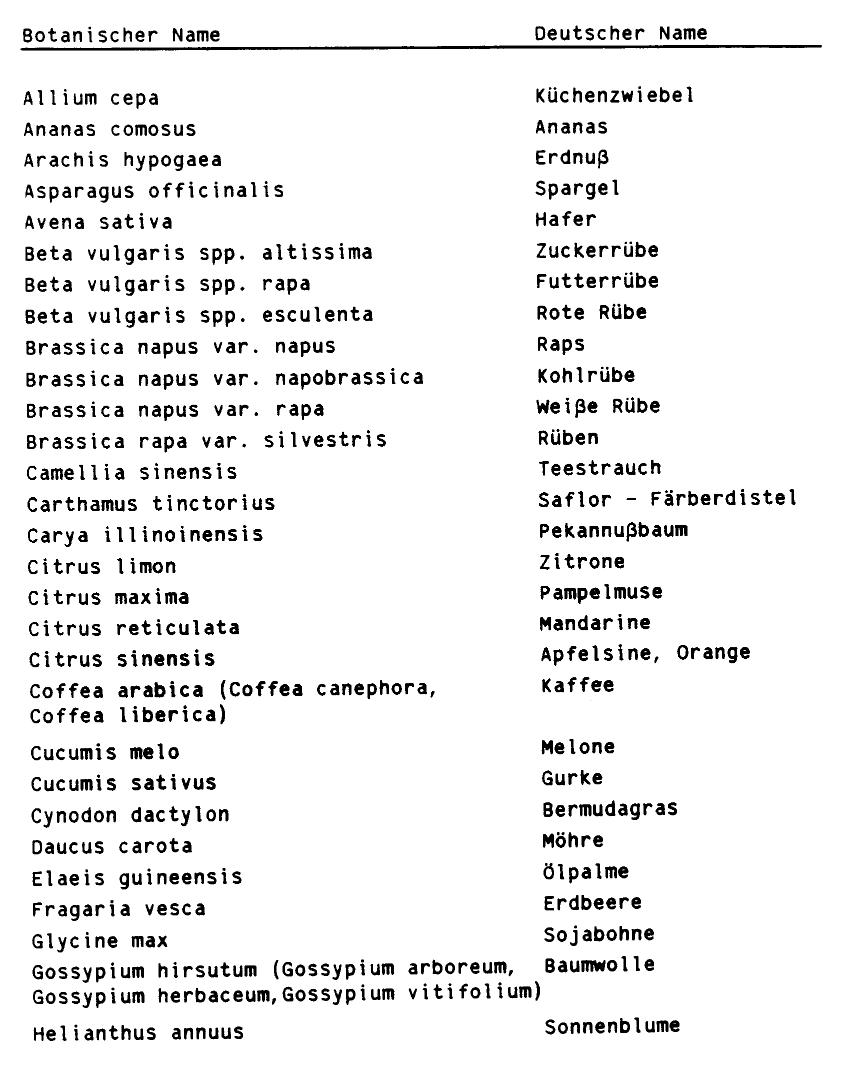

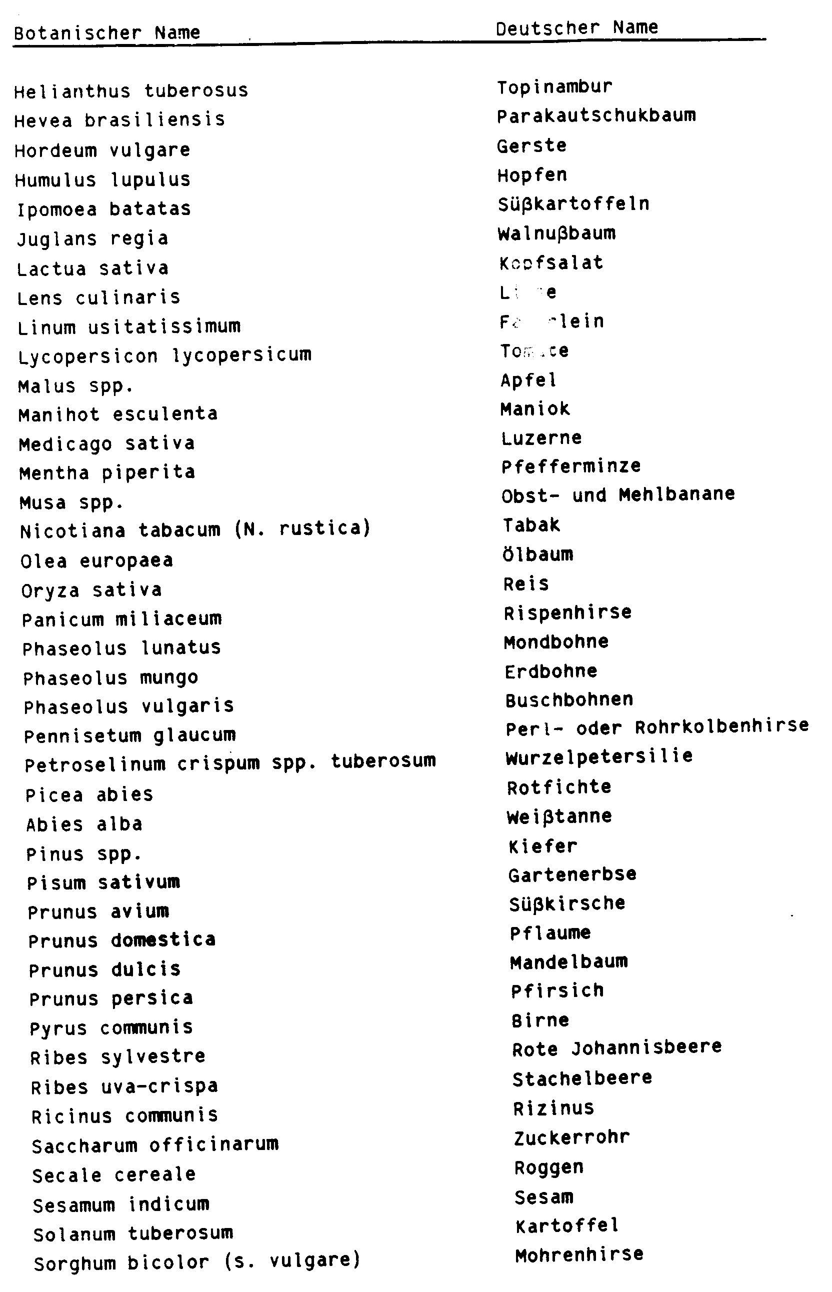

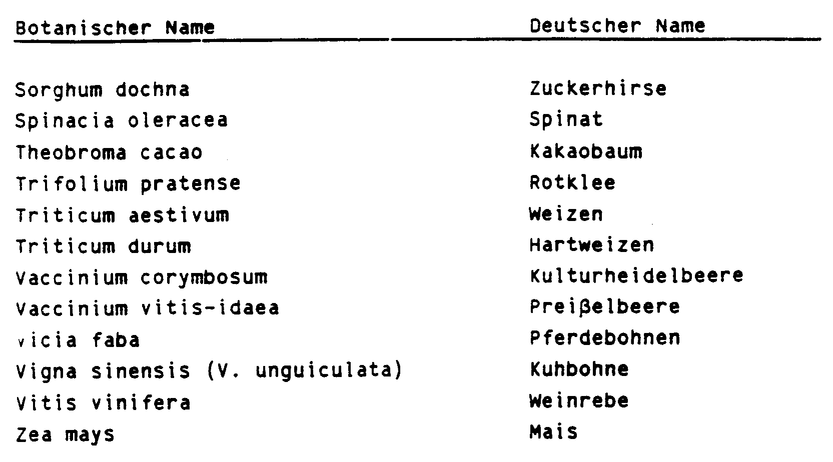

Zur Verbreiterung des Wirkungsspektrums und zur Erzielung synergistischer Effekte können die N-Phenyltetrahydroindazole der Formel Ia und Ib mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Chinolincarbonsäurederivate, Phenyloxy- bzw. Heteroaryloxy-phenylpropionsäuren sowie deren Salze, Ester und Amide und andere in Betracht.

Außerdem kann es von Nutzen sein, die N-Phenyltetrahydroindazole der Formel Ia und Ib allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit alt Mineralsalzlösungen, welche zur Behebung von Ernährungs-und Spurenelementmängeln eingesetzt werden. Es können auch nichtphytotoxische Öle und Ölkonzentrate zugesetzt werden.

### Synthesebeispiele:

Die in den nachstehenden Sythesebeispielen wiedergegebenen Vorschriften wurden unter entsprechender Abwandlung der Ausgangsverbindungen zur Gewinnung weiterer Verbindungen der Formel Ia und Ib benutzt; die erhaltenen Verbindungen sind in den nachfolgenden Tabellen mit physikalischen Angaben aufgeführt; Verbindungen ohne diese Angaben lassen sich aus den entsprechenden Stoffen in analoger Weise aufbauen. Sie lassen aufgrund ihrer nahen strukturellen Beziehungen zu den hergestellten und untersuchten Verbindungen eine gleichartige Wirkung erwarten.

### Beispiel 1

### Herstellung von 3-Chlor-2-[4-chlor-2-fluor-5-(5.6-dihydro-2H-pyran-3-yl-methyloxy)-phenyl]-4.5.6.7.-tetrahydroindazol

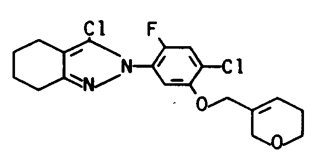

Zu einer Mischung von 4,5 g (15 mmol) 3-Chlor-2-(4-chlor-2-fluor-5-hydroxyphenyl)-4.5.6.7.-tetrahydroindazol, 2,5 g (18 mmol) Kaliumcarbonat und 0,5 g (3 mmol) Natriumiodid in 50 ml Dimethylformamid wurden bei 25°C 2,1 g (16 mmol) 3-Chlormethyl-5.6-dihydro-2H-pyran gegeben und 12 Stunden gerührt. Man versetzte die Reaktionsmischung mit 100 ml Wasser und isolierte den ausgefallenen Niederschlag. Nach Waschen mit Wasser und Trocknen erhielt man 5 g (84 %) der Titelverbindung [Fp. 90 bis 92°C]. Verbindung Nr. 1.002.

### Beispiel 2

### Herstellung von 3-Chlor-2-[4-chlor-2-fluor-5-(hexahydrothiopyran-3-yl-methyloxy)-phenyl]-4.5.6.7.-tetrahydroindazol

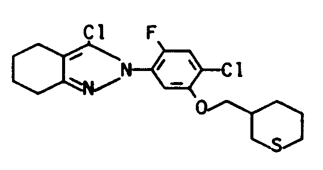

a) 47,9 g (250 mmol) 2-Chlor-4-fluor-5-nitrophenol, 34,6 g (250 mmol) Kaliumcarbonat, 1 g Kaliumiodid und 58,5 g (300 mmol) 3-Brommethyltetrahydrothiopyran wurden in 500 ml Acetonitril 5 Stunden unter Rückfluß erhitzt. Nach dem Abkühlen auf 25°C wurde abfiltriert, das Filtrat von Lösungsmittel befreit und der Rückstand in Methylenchlorid aufgenommen, mit Wasser gewaschen, getrocknet und eingeengt. Man erhielt 22 g (29 %) 2-Chlor-4-fluor-5-nitro-(tetrahydrothiopyran-3-yl-methyloxy)benzol (Fp. 100 bis 103°C ).
b) Zu einer Mischung von 14,3 g (260 mmol) Eisenpulver in 100 ml Methanol und 28 ml Eisessig gab man bei Siedetemperatur eine Suspension aus 26 g (85 mmol) der obigen Nitroverbindung und beließ weitere 2 Stunden am Rückfluß. Nach dem Abkühlen auf 25°C wurde das Reaktionsgemisch in Wasser gegeben, Essigsäureethylester extrahiert, getrocknet und eingeengt. Nach Reinigung erhielt man 15,5 g (66 %) 4-Chlor-2-fluor-5-(Tetrahydrothiopyran-3-yl-methyloxy)anilin (Fp. 66 bis 68°C).
c) Zu einer Lösung von 13,8 g (50 mmol) 4-Chlor-2-fluor-5-(tetrahydrothiopyran-3-yl-methyloxy)-anilin in 50 ml Eisessig wurden 100 ml 10 %ige Salzsäure gegeben und anschließend bei -5 bis 0°C innerhalb 15 min 3,5 g (50 mmol) Natriumnitrit in 20 ml Wasser versetzt.
   Nach 1 Stunde Rühren bei dieser Temperatur wurde die so gewonnene Lösung bei -10 bis -5°C zu 28,2 g (125 mmol) Zinn-(II)-chlorid-dihydrat in 30 ml konzentrierter Salzsäure getropft, weitere 2 Stunden bei 0°C gerührt. Unter Eiskühlung wurde mit konzentrierter Natronlauge alkalisch gestellt, Methylenchlorid extrahiert, die organischen Phasen getrocknet, eingeengt und mit Petrolether verrührt. Man erhielt 12 g (82 %) 4-Chlor-2-fluor-5-(tetrahydrothiopyran-3-yl-methyloxy)-phenylhydrazin (Fp. 83 bis 87°C).
d) 7,3 g (25 mmol) des so gewonnenen Phenylhydrazins wurden zu 4,3 g (25 mmol) Cyclohexanon-2-carbonsäureethylester in 100 ml Eisessig gegeben und 5 Stunden unter Rückfluß erhitzt. Nach dem Abkühlen auf 25°C wurde eingeengt, durch Zugabe von wenig Wasser/Petrolether ausgefällt und der Rückstand getrocknet. Man erhielt 9 g (90 %) 2-[4-Chlor-2-fluor-5-(tetrahydrothiopyran-3-yl-methyloxy)-phenyl]-1.2.4.5.6.7-hexahydro-3H-indazol-3-on (Fp. 96 bis 100°C ).
e) Zu einer Lösung von 30 ml Chlorameisensäuretrichlormethylester in 500 ml Toluol wurden 8,7 g (22 mmol) des obigen Indazol-3-on gegeben und langsam aufgeheizt. Nach dem Abkühlen auf 25°C wurde mit Stickstoff gespült, eingeengt und wie üblich gereinigt. Man erhielt 2 g (22 %) der Titelverbindung als Öl (Verbindung Nr. 1.005).

### Anwendungsbeispiele

Die Wirkung der N-(Phenyl)tetrahydroindazolderivate I und Ib auf das Wachstum der Testpflanzen läßt sich durch folgende Gewächshausversuche zeigen:
Zur Anzucht der Testpflanzen dienen Plastikblumentöpfe mit 300 cm³ Inhalt und lehmigem Sand mit etwa 3,0 % Humus als Substrat. Die Samen der Testpflanzen werden nach Arten getrennt eingesät.

Zum Zweck der Nachauflaufbehandlung werden entweder direkt gesäte oder in den gleichen Gefäßen aufgewachsene Pflanzen ausgewählt oder sie werden erst als Keimpflanzen getrennt angezogen und einige Tage vor der Behandlung in die Versuchsgefäße verpflanzt.

Je nach Wuchsform werden die Testpflanzen bei einer Wuchshöhe von 3 bis 15 cm dann mit den in Wasser als Verteilungsmittel suspendierten oder emulgierten Wirkstoffen, die durch fein verteilende Düsen gespritzt werden, behandelt. Die Aufwandmenge für die Nachauflaufbehandlung beträgt 0,03 kg/ha a.S..

Die Versuchsgefäße werden im Gewächshaus aufgestellt, wobei für wärmeliebende Arten wärmere Bereiche (20 bis 35°C) und für solche gemäßigter Klimate 10 bis 20°C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet.

Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzen sich aus folgenden Arten zusammen:

| Lateinischer Name | Deutscher Name |
|---|---|
| Abutilon theophrasti | Chinesischer Hanf |
| Amaranthus retroflexus | Zurückgekrümmter Fuchsschwanz |
| Chenopodium album | Weißer Gänsefuß |
| Chrysanthemum corinarium | Kronenwucherblume |
| Galium aparine | Klettenlabkraut |
| Ipomoea spp. | Prunkwindearten |
| Lamium amplexicaule | Stengelumfassende Taubnessel |
| Malva neglecta | Wegmalve |
| Solanum nigrum | Schwarzer Nachschatten |
| Stellaria media | Vogelsternmiere |

Mit 0,03 kg/ha a.S. in Nachauflaufverfahren eingesetzt, lassen sich mit den Beispielen 1.005, 1.003, 1.001 und 1.002 breitblättrige unerwünschte Pflanzen sehr gut bekämpfen.

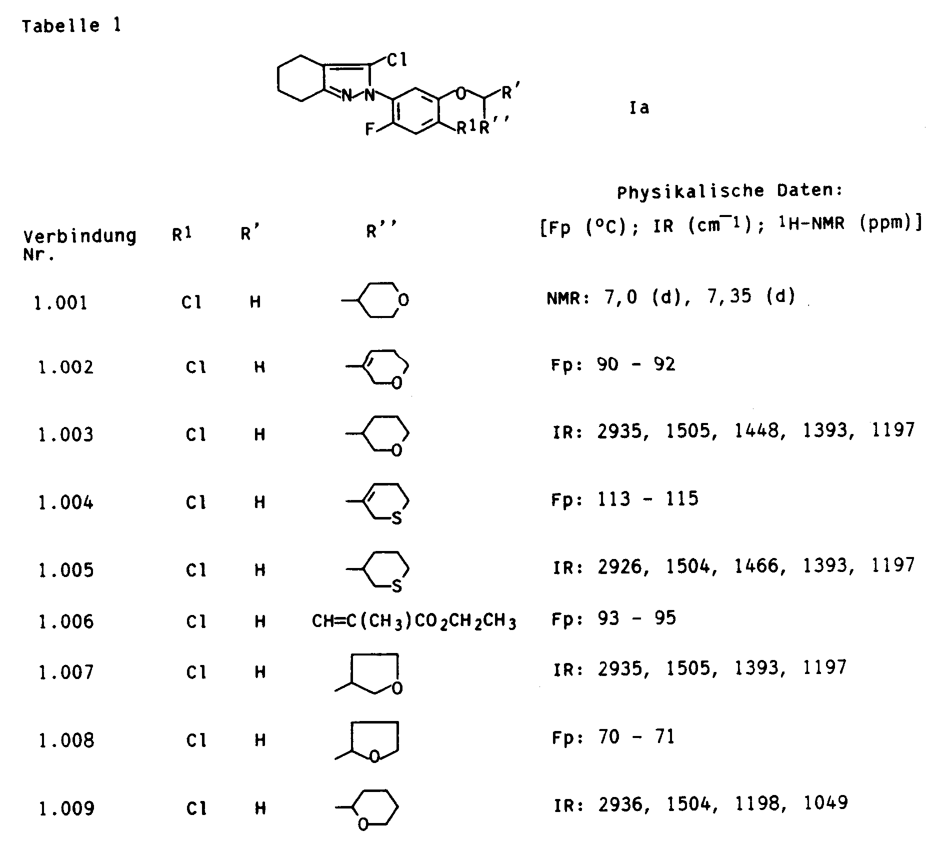

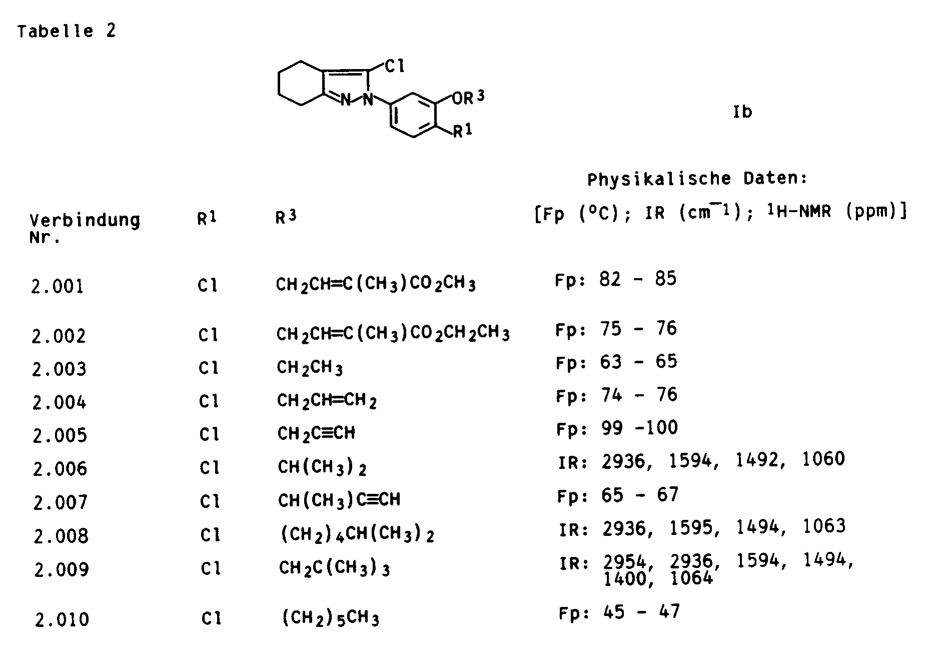

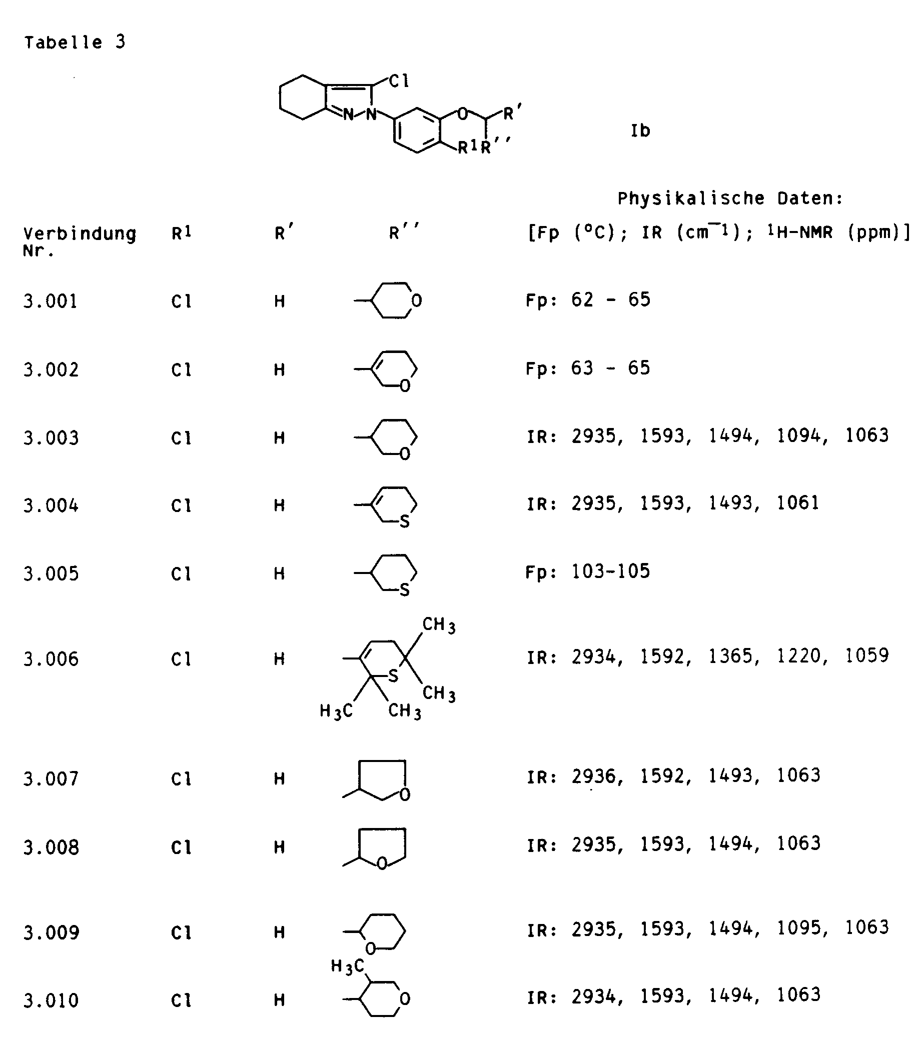

## Patentansprüche

1. N-(Phenyl)tetrahydroindazolderivate der allgemeinen Formeln Ia und Ib 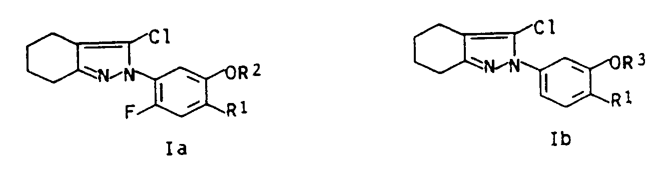 in denen die Substituenten folgende Bedeutung haben:
R¹ Halogen;
R² eine C₁-C₆-Alkoxycarbonyl-C₃-C₅-alkenylgruppe oder eine C₁-C₃-Alkylgruppe, welche in 1- oder 2-Position durch einen 5- oder 6-gliedrigen gesättigten oder einfach ungesättigten Heterocyclus substituiert ist, wobei dieser Heterocyclus ein Sauerstoff- oder Schwefelatom enthält und durch ein bis vier C₁-C₄-Alkylgruppen substituiert sein kann, und
R³ eine C₂-C₇-Alkylgruppe, eine C₃-C₆-Alkenylgruppe, eine C₃-C₆-Alkinylgruppe, eine C₁-C₆-Alkoxycarbonyl-C₃-C₅-alkenylgruppe oder eine C₁-C₃-Alkylgruppe, welche in 1- oder 2-Position durch eine C₁-C₆-Alkoxcarbonylgruppe oder einen 5- oder 6-gliedrigen gesättigten oder einfach ungesättigten Heterocyclus substituiert ist, wobei dieser Heterocyclus ein Sauerstoff- oder Schwefelatom enthält und durch ein bis vier C₁-C₄-Alkylgruppen substituiert sein kann.

2. Verfahren zur Herstellung der Verbindungen Ia gemäß Anspruch 1, dadurch gekennzeichnet, daß man ein N-(Hydroxyphenyl)-indazolderivat der Formel IIa 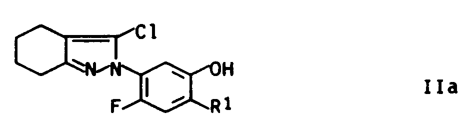 in an sich bekannter Weise bei Temperaturen bis 200°C in Gegenwart einer Base in einem inerten organischen Lösungsmittel mit einer Verbindung IIIa,
Nu-R²,
worin Nu eine übliche nucleophile Abgangsgruppe bedeutet, zum Derivat Ia verethert.

3. Verfahren zur Herstellung der Verbindungen Ia gemäß Anspruch 1, dadurch gekennzeichnet, daß man ein Nitrophenolderivat IVa 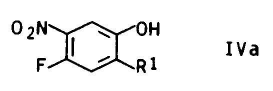 in an sich bekannter Weise zunächst bei Temperaturen bis 200°C in einem aprotisch polaren Lösungsmittel in Gegenwart einer Base mit einer Verbindung IIIa,
Nu-R² IIIa
gemäß Anspruch 2, in der Nu eine übliche nucleophile Abgangsgruppe bedeutet zum Nitrophenylether Va 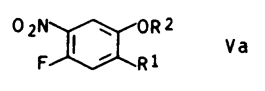 verethert, anschließend nach üblichen Methoden mit einem anorganischen Reduktionsmittel in einem inerten organischen Lösungsmittel zum Anilinderivat VIa 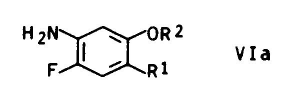 reduziert, dieses in an sich bekannter Weise in einem inerten Lösungsmittel diazotiert und in situ mittels eines anorganischen Reduktionsmittel zum Phenylhydrazin VIIa 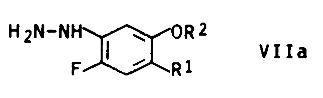 reduziert, dieses anschließend in einem inerten Lösungsmittel bei Temperaturen bis 200°C in an sich bekannter weise mit einem Cyclohexanon-2-carbonsäurederivat VIII, 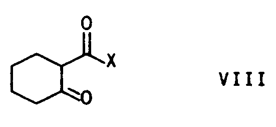 worin X eine übliche nucelophile Abgangsgruppe bedeutet, zum Indazolderivat IXa 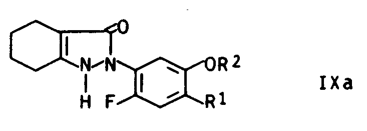 cyclisiert und daraufhin bei Temperaturen bis 200°C in einem inerten organischen Lösungsmittel in an sich bekannter Weise mit einem allgemein üblichen Chlorierungsmittel in das N-(Phenyl)-tetrahydroindazolderivat Ia überführt.

4. Verfahren zur Herstellung von Verbindungen Ib gemäß Anspruch 1, dadurch gekennzeichnet, daß man analog den in Anspruch 3 geschilderten Bedingungen ein Nitrophenolderivat IVb 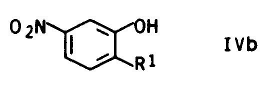 zunächst mit einer Verbindung IIIb,
Nu-R³ IIIb
in der Nu eine übliche nucleophile Abgangsgruppe bedeutet, zum Nitrophenylether Vb 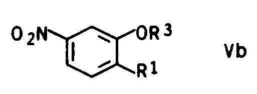 verethert, anschließend zum Anilinderivat VIb 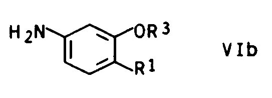 reduziert, dieses mit einem Cyclohexanoncarbonsäurederivat VIII 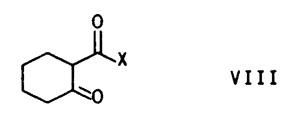 gemäß Anspruch 3, worin X die dort gegebene Bedeutung hat, zum Indazolderivat IXb 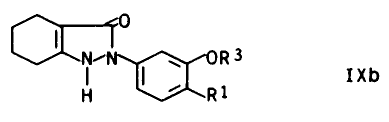 cyclisiert und daraufhin mit einem allgemein üblichen Chlorierungsmittel in an sich bekannter Weise in das N-(Phenyl)tetrahydroindazolderivat Ib überführt.

5. Verfahren zur Herstellung von Verbindungen der Formel Ib gemäß Anspruch 1, dadurch gekennzeichnet, daß man ein N-(Phenyl)tetrahydroindazolderivat Ic 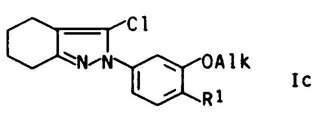 in dem R³ C₁-C₄-Alkyl(Alk) bedeutet, in an sich bekannter Weise in einem inerten organischen Lösungsmittel bei Temperaturen bis 150°C mit einer üblichen Lewissäure zum N-(Hydroxyphenyl)tetrahydroindazolderivat IIb 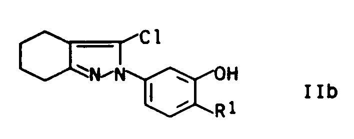 spaltet und dieses anschließend in an sich bekannter Weise analog zu den in Anspruch 2 geschilderten Bedingungen mit einer Verbindung IIIb
Nu-R³ IIIb,
worin Nu die in Anspruch 2 gegebene Bedeutung hat, zum N-(Phenyl)tetrahydroindazolderivat Ib verethert.

6. Verwendung der N-(Phenyl)tetrahydroindazolderivate der Formeln Ia und Ib gemäß Anspruch 1 als Herbizide.

7. Herbizide Mittel, enthaltend ein N-(Phenyl)tetrahydroindazolderivat der Formel Ia und/oder Ib gemäß Anspruch 1 und inerte Zusatzstoffe.

8. Herbizide Mittel gemäß Anspruch 7, enthaltend ein N-(Phenyl)tetrahydroindazolderivat der Formeln Ia und/oder Ib und weitere wirksame Bestandteile.

9. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines N-(Phenyl)tetrahydroindazolderivates Ia und/oder Ib gemäß Anspruch 1 behandelt.

## Claims

1. N-phenyltetrahydroindazole derivatives of the general formulae Ia and Ib 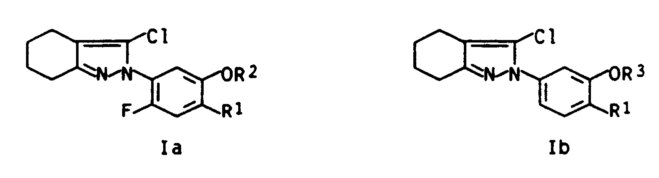 where
R¹ is halogen;
R² is C₁-C₆-alkoxycarbonyl-C₃-C₅-alkenyl or C₁-C₃-alkyl which is substituted in the 1- or 2-position by a 5- or 6-membered saturated or monosaturated heterocycle which contains one oxygen or sulfur and can be substituted by one to four C₁-C₄-alkyls, and
R³ is C₂-C₇-alkyl, C₃-C₆-alkenyl, C₃-C₆-alkynyl, C₁-C₆-alkoxy-carbonyl-C₃-C₅-alkenyl or C₁-C₃-alkyl which is substituted in the 1- or 2- position by C₁-C₆-alkoxycarbonyl or a 5- or 6-membered saturated or monosaturated heterocycle which contains one oxygen or sulfur and can be substituted by one to four C₁-C₄-alkyls.

2. A process for the preparation of compounds Ia as claimed in claim 1, wherein an N-(hydroxyphenyl)indazole derivative of the formula IIa 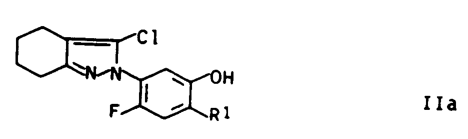 is etherified in a conventional manner at up to 200°C in the presence of a base in an inert organic solvent with a compound IIIa
Nu-R²,
where Nu is a conventional nucleophilic leaving group, to give the derivative Ia.

3. A process for the preparation of compounds Ia as claimed in claim 1, wherein a nitrophenol derivative IVa 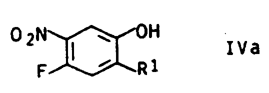 is etherified in a conventional manner initially at up to 200°C in an aprotic polar solvent in the presence of a base with a compound IIIa
Nu-R² IIIa
as claimed in claim 2, where Nu is a conventional nucleophilic leaving group, to give the nitrophenyl ether Va 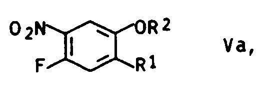 which is then reduced by conventional methods with an inorganic reducing agent in an inert organic solvent to give the aniline derivative VIa 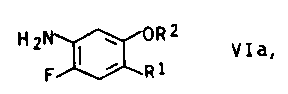 which is then diazotized in a conventional manner in an inert solvent and reduced in situ by means of an inorganic reducing agent to give the phenylhydrazine VIIa 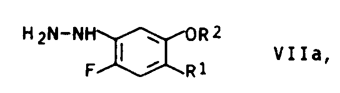 which is then cyclized in an inert solvent at up to 200°C in a conventional manner with a cyclohexanone-2-carboxylic acid derivative VIII 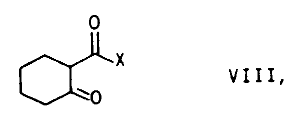 where X is a conventional nucleophilic leaving group, to give the indazole derivative IXa 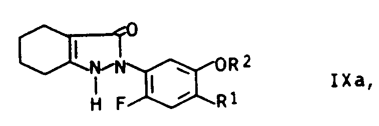 which is then converted at up to 200°C in an inert organic solvent in a conventional manner with a conventional chlorinating agent to give the N-phenyltetrahydroindazole derivative Ia.

4. A process for the preparation of compounds Ib as claimed in claim 1, wherein analogously to the conditions described in claim 3, a nitrophenol derivative IVb 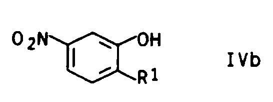 is initially etherified with a compound IIIb
Nu-R³ IIIb,
where Nu is a conventional nucleophilic leaving group, to give the nitrophenyl ether Vb 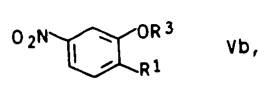 which is then reduced to the aniline derivative VIb 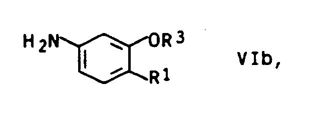 which is cyclized with a cyclohexanonecarboxylic acid derivative VIII 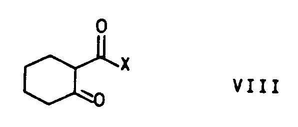 as in claim 3, where X has the meanings given therein, to give the indazole derivative IXb 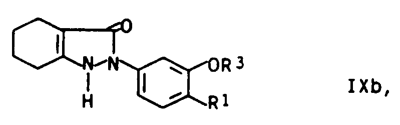 which is then converted with a conventional chlorinating agent in a conventional manner to give the N-phenyltetrahydroindazole derivative Ib.

5. A process for the preparation of compounds of the formula Ib as claimed in claim 1, wherein an N-phenyltetrahydroindazole derivative Ic 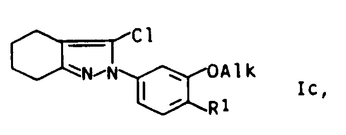 where R³ is C₁-C₄-alkyl(alk), is cleaved in a conventional manner in an inert organic solvent at up to 150°C with a conventional Lewis acid to give the N-hydroxyphenyltetrahydroindazole derivative IIb 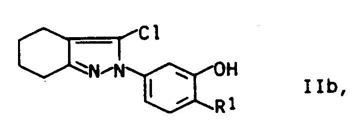 which is then etherified in a conventional manner analogously to the conditions described in claim 2 with a compound IIIb
Nu-R³ IIIb,
where Nu has the meanings given in claim 2, to give the N-phenyltetrahydroindazole derivative Ib.

6. The use of N-phenyltetrahydroindazole derivatives of the formulae Ia and Ib as claimed in claim 1 as herbicides.

7. Herbicidal agents containing an N-phenyltetrahydroindazole derivative of the formula Ia and/or Ib as claimed in claim 1, and inert additives.

8. Herbicidal agents as claimed in claim 7, containing an N-phenyltetrahydroindazole derivative of the formulae Ia and/or Ib and further active constituents.

9. A method for controlling undesired plant growth, wherein the undesired plants and/or their habitat are treated with a herbicidally effective amount of an N-phenyltetrahydroindazole derivative Ia and/or Ib as claimed in claim 1.

## Revendications

1. Dérivés de N-(phényl)-tétrahydroindazoles de formules générales Ia et Ib 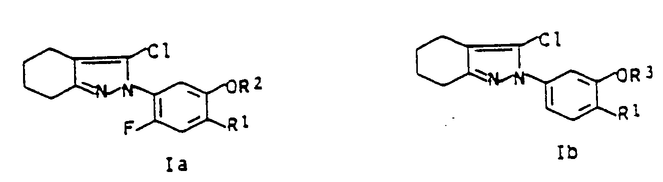 dans lesquelles les symboles ont les significations suivantes
R¹ représente un halogène;
R² représente un groupe (alcoxy en C1-C6)-carbonyl-alcényle en C3-C5 ou un groupe alkyle en C1-C3 substitué en position 1 ou 2 par un hétérocycle saturé ou mono-insaturé à 5 ou 6 chaînons, cet hétérocycle contenant un atome d'oxygène ou de soufre et pouvant porter 1 à 4 substituants alkyle en C1-C4, et
R³ représente un groupe alkyle en C2-C7, un groupe alcényle en C3-C6, un groupe alcynyle en C3-C6, un groupe (alcoxy en C1-C6)-carbonyl-alcényle en C3-C5 ou un groupe alkyle en C1-C3 qui est substitué en position 1 ou 2 par un groupe (alcoxy en C1-C6)-carbonyle ou un hétérocycle saturé ou mono-insaturé à 5 ou 6 chaînons, cet hétérocycle contenant un atome d'oxygène ou de soufre et pouvant porter 1 à 4 substituants alkyle en C1-C4.

2. Procédé de préparation des composés Ia de la revendication 1, caractérisé en ce que l'on éthérifie en le composé Ia un dérivé de N-(hydroxyphényl)-indazole de formule IIa 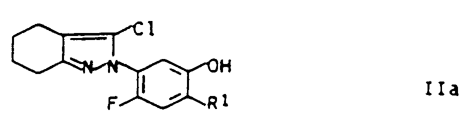 de manière connue en soi, à des températures allant jusqu'à 200°C, en présence d'une base, dans un solvant organique inerte, à l'aide d'un composé IIIa
Nu-R²,
dans laquelle Nu représente un groupe nucléophile éliminable usuel.

3. Procédé de préparation des composés Ia de la revendication 1, caractérisé en ce que l'on éthérifie d'abord un dérivé de nitrophénol IVa 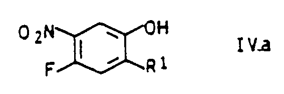 de manière connue en soi, à des températures allant jusqu'à 200°C, dans un solvant polaire aprotonique, en présence d'une base, à l'aide d'un composé IIIa
Nu-R² IIIa
selon la revendication 2, dans lequel Nu représente un groupe nucléophile éliminable usuel, ce qui donne l'éther nitrophénylique Va 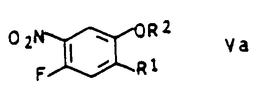 qu'on réduit ensuite, par des modes opératoires usuels, à l'aide d'un agent réducteur inorganique, dans un solvant organique inerte, en le dérivé d'aniline VIa 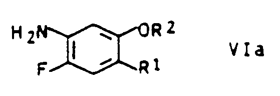 qu'on diazote de manière connue en soi dans un solvant inerte, le diazo étant réduit in situ à l'aide d'un agent réducteur inorganique, en la phénylhydrazine VIIa 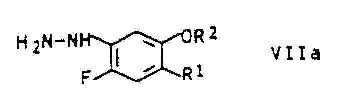 qu'on cyclise ensuite dans un solvant inerte, à des températures allant jusqu'à 200°C, de manière connue en soi, à l'aide d'un dérivé d'acide cyclohexanone-2-carboxylique VIII 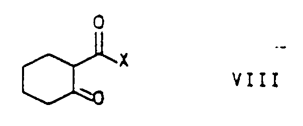 dans lequel X représente un groupe nucléophile éliminable usuel, en le dérivé d'indazole IXa 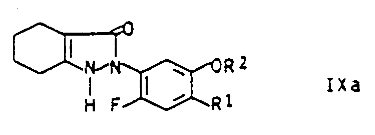 qu'on convertit lui-même, à des températures allant jusqu'à 200°C, dans un solvant organique inerte, de manière connue en soi, à l'aide d'un agent chlorant usuel, en le dérivé de N-(phényl)-tétrahydroindazole Ia.

4. Procédé de préparation des composés Ib de la revendication 1, caractérisé en ce que, dans des conditions analogues à celles décrites dans la revendication 3, on éthérifie d'abord un dérivé de nitrophénol IVb 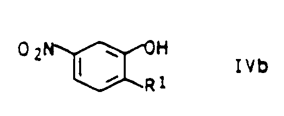 à l'aide d'un composé IIIb
Nu-R³ IIIb
dans lequel Nu représente un groupe nucléophile éliminable usuel, en l'éther nitrophénylique Vb 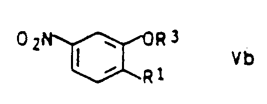 qu'on réduit ensuite en le dérivé d'aniline VIb 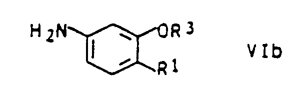 qu'on cyclise à l'aide d'un dérivé d'acide cyclohexanone-carboxylique VIII 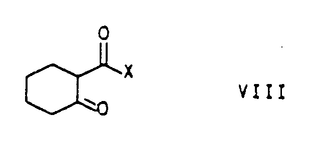 selon la revendication 3, dans lequel X a les significations indiquées dans la revendication 3, en le dérivé d'indazole IXb 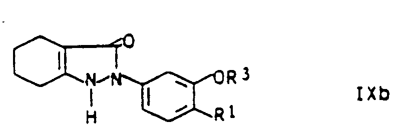 qu'on convertit ensuite, de manière connue en soi, à l'aide d'un agent chlorant usuel en le dérivé de N-(phényl)-tétrahydroindazole Ib.

5. Procédé de préparation des composés de formule Ib selon la revendication 1, caractérisé en ce que l'on scinde un dérivé de N-(phényl)-tétrahydroindazole Ic 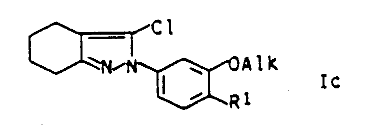 dans laquelle R³ représente un groupe alkyle en C1-C4 (Alk), de manière connue en soi, dans un solvant organique inerte à des températures allant jusqu'à 150°C, à l'aide d'un acide de Lewis usuel, en le dérivé de N-(hydroxyphényl)-tétrahydroindazole IIb 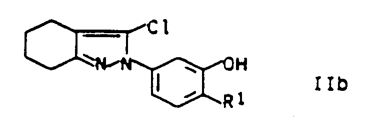 qu'on éthérifie ensuite de manière connue en soi, dans des conditions analogues à celles décrites dans la revendication 2, à l'aide d'un composé IIIb
Nu-R³ IIIb
dans lequel Nu a les significations indiquées dans la revendication 2, en le dérivé de N-(phényl)-tétrahydroindazole Ib.

6. Utilisation des dérivés de N-(phényl)-tétrahydroindazoles de formules Ia et Ib selon la revendication 1 en tant qu'herbicides.

7. Produits herbicides contenant un dérivé de N-(phényl)-tétrahydroindazole de formule Ia et/ou Ib selon la revendication 1 et des additifs inertes.

8. Produits herbicides selon la revendication 7, contenant un dérivé de N-(phényl)-tétrahydroindazole de formule Ia et/ou Ib et d'autres composants actifs.

9. Procédé pour combattre les croissances de végétaux indésirables, caractérisé en ce que l'on traite les végétaux indésirables et/ou leur habitat par une quantité herbicide efficace d'un dérivé de N-(phényl)-tétrahydroindazole Ia et/ou Ib selon la revendication 1.
